# FASCICULE DE BREVET EUROPEEN

(11) **EP 1 744 719 B1**
(45) Date de publication et mention de la délivrance du brevet: **25.03.2009**
(21) Numéro de dépôt: 05763583.1
(22) Date de dépôt: 28.04.2005
(51) Int. Cl.: A61K 6/093, C07D 335/16

(54) **COMPOSITION DENTAIRE PHOTOPOLYMERISABLE**
FOTOPOLYMERISIERBARE DENTALE ZUSAMMENSETZUNGEN
PHOTOPOLYMERISABLE DENTAL COMPOSITION

(30) Priorité: 13.05.2004 FR 0405176; 30.06.2004 FR 0407210
(43) Date de publication de la demande: 24.01.2007
(73) Titulaire: Bluestar Silicones France, 69486 Lyon Cedex 03 (FR)
(72) Inventeur: FRANCES, Jean-Marc, F-69330 Meyzieu (FR)
(74) Mandataire: Valentino, Cédric
(86) Numéro de dépôt international: PCT/FR2005/001049
(87) Numéro de publication internationale: WO 2005/120439

(56) Documents cités:
- EP-A- 1 203 999
- FR-A- 2 784 025
- US-A- 4 791 213
- PATENT ABSTRACTS OF JAPAN vol. 2000, no. 24, 11 mai 2001 (2001-05-11) -& JP 2001 206903 A (TOYO INK MFG CO LTD), 31 juillet 2001 (2001-07-31)
- PATENT ABSTRACTS OF JAPAN vol. 0183, no. 28 (C-1215), 22 juin 1994 (1994-06-22) -& JP 06 073306 A (TOSOH CORP), 15 mars 1994 (1994-03-15)
- EISELE G ET AL: "LE ROLE DES SYSTèMES PHOTOAMORCEURS DANS L'EFFICACITé DE PHOTORéTICULATION DE L'HOMOPOLYMèRE DICYCLOPENTADIèNE ACRYLATEA (DCPA) ET ACRYLATE D'éTHYLE (AE) ET DES DéRIVéS COPOLYMèRES DCPA/AE" ANGEWANDTE MAKROMOLEKULARE CHEMIE. APPLIED MACROMOLECULAR CHEMISTRY AND PHYSICS, WILEY VCH,WEINHEIM, DE, vol. 239, 1 juillet 1996 (1996-07-01), pages 169-190, XP000627572 ISSN: 0003-3146

## Description

Le domaine de l'invention est celui des compositions dentaires. Plus précisément, les compositions dentaires mises au point dans le cadre de la présente invention sont utilisables pour la réalisation de prothèses dentaires et pour la restauration dentaire.

Ces compositions dentaires sont classiquement des résines époxy, ou silicones photopolymérisables ou des résines acrylates polymérisables par voie radicalaire. Ces compositions incluent en outre des charges particulaires de renfort (e.g. en silice hydrophobisée), des photoamorceurs et éventuellement des photosensibilisateurs voire d'autres additifs fonctionnels tels que des pigments ou des stabilisants.

Une fois mélangées, ces compositions sont mises en forme puis photoréticulées en une masse de structure analogue à celle des dents.

Par exemple, la demande de brevet FR-A-2 784 025 décrit des compositions dentaires à base de résines silicones polymérisables/réticulables, par voie cationique et sous irradiation suivie ou non d'une post-réticulation thermique. Ces résines silicones sont à fonctionnalités oxirane (époxyde, oxétane...) ou vinyléther. De telles compositions comprennent :
- un ou plusieurs polydiméthylsiloxanes réticulables et/ou polymérisables par voie cationique et porteurs à au moins l'une de leurs extrémités des fonctions réactives de formule : 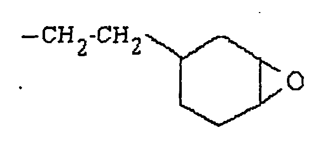
- une quantité efficace d'au moins un amorceur de type borate d'onium: 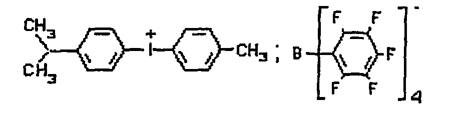
- au moins un photosensibilisateur, et
- au moins une charge dentaire ou de renforcement inerte à base de verres dentaire polyméthacrylate de méthyle ou de silice de combustion éventuellement traitée hexaméthyldisilazane ou polydiméthylsiloxane de surface spécifique 200 m²/g..

Ces compositions dentaires sont destinées à la fabrication de prothèses ou d'appareils dentaires et à la restauration dentaire.

Ces silicones présentent l'avantage par rapport à des résines organiques réticulant par voie cationique d'être très transparents à la lumière UV-visible et donc de permettre l'obtention de matériaux très épais (plusieurs millimètres d'épaisseur) photoréticulés en très peu de temps (inférieur à une minute) avec une lampe UV émettant dans le domaine du visible >400 nm.

Cependant ces silicones sont formulés avec des sels d'iodonium et des photosensibilisateurs, en particulier des thioxanthones, qui engendrent une grande variation chromatique lors de l'exposition à des rayonnements de longueur d'onde supérieure à 390 nm lors de la photoréticulation. Ceci se traduit par une coloration rosâtre du produit fini (après exposition) qui n'est pas souhaitable sur le plan esthétique.
De plus, un autre problème résultant de l'utilisation de photosensibilisateurs concerne une cinétique de réticulation insuffisante lorsque des matériaux très épais (plusieurs millimètres d'épaisseur) sont préparés.

Il apparaît donc que l'art antérieur n'apporte pas de solution satisfaisante au double problème de la faible stabilité chromatique des produits dentaires finis (après réticulation) et de la cinétique de réticulation insuffisante lorsque des matériaux très épais (plusieurs millimètres d'épaisseur) sont préparés.

L'un des objectifs essentiels de la présente invention est donc de remédier à cela en fournissant de nouvelles compositions dentaires photopolymérisables en particulier à base de motifs polymérisables par voie cationique sous UV (par exemple des oxiranes), ne présentant pas les inconvénients de l'art antérieur.

Ces objectifs, parmi d'autres, sont atteints par la présente invention qui concerne tout d'abord une composition dentaire photopolymérisable par un rayonnement de longueur d'onde supérieure à 390 nm comprenant :
(1) au moins un composé **(A)** réactif par voie cationique ;
(2) au moins une charge dentaire **(B);**
(3) éventuellement au moins un dispersant **(C)** comprenant au moins un polymère ou copolymère organique ;
(3) au moins un photoamorceur cationique **(D) ;** et
(4) au moins un photosensibilisateur **(E)** qui est un sel d'une thioxanthone substituée par au moins un groupement G comprenant une fonction ammonium
ledit photosensibilisateur **(E),** éventuellement en association avec au moins une camphorquinone, une phénanthrènequinone et/ou un anthracène substitué, a pour formule: 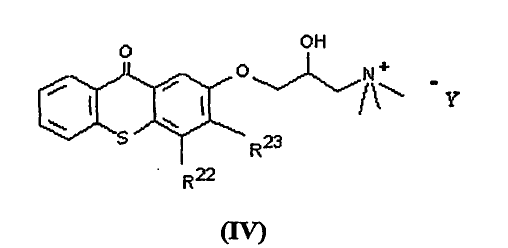 formule dans laquelle :
- R²² et R²³ sont identiques ou différents et représentent un hydrogène ou un radical alkyle en C1-C10, éventuellement substitué, de préférence R²² = R²³ = méthyle,
- (Y⁻) étant une entité anionique choisie parmi le groupe constitué par : BF₄⁻, PF₆⁻; SbF₆⁻; l'anion **(I)** de formule [BXₐ R_{b}]⁻ défini ci-dessous, R_{f}SO₃⁻ ; (R_{f}SO₂)₃C⁻ ou (R_{f}SO₂)₂N⁻, avec R_{f} étant un radical alkyle linéaire ou ramifié, substitué par au moins un atome d'halogène, préférentiellement un atome de fluor,
   et encore plus préférentiellement (Y⁻) est choisi parmi les borates de formules suivantes : [B(C₆H₃(CF₃)₂)₄]⁻ et [B(C₆F₅)₄]⁻;
   ledit anion (I) de formule [BXₐ R_{b}]⁻ étant défini de la manière suivante :

- a et b sont des nombres entiers allant pour a de 0 à 3 et pour b de 1 à 4 avec a+b=4,
- les symboles X représentent :
   * un atome d'halogène (chlore, fluor) avec a = 0 à 3, ou
   * une fonction OH avec a = 0 à 2,
- les symboles R sont identiques ou différents et représentent :
   - un radical phényle substitué par au moins un groupement électroattracteur tel que par exemple OCF₃, CF₃, NO₂, CN, et/ou par au moins 2 atomes d'halogène (fluor tout particulièrement), et ce lorsque l'entité cationique est un onium d'un élément des groupes 15 à 17,
   - un radical phényle substitué par au moins un élément ou un groupement électroattracteur notamment atome d'halogène (fluor tout particulièrement), CF₃, OCF₃, NO₂, CN, et ce lorsque l'entité cationique est un complexe organométallique d'un élément des groupes 4 à 10, ou
   - un radical aryle contenant au moins deux noyaux aromatiques tel que par exemple biphényle, naphtyle, éventuellement substitué par au moins un élément ou un groupement électroattracteur, notamment un atome d'halogène dont le fluor en particulier, OCF₃, CF₃, NO_{2,} CN, quelle que soit l'entité cationique.

Il est du mérite des inventeurs d'avoir montré, de manière surprenante et inattendue qu'une certaine classe de thioxanthone permet de remédier aux problèmes liés à l'utilisation de photosensibilisateurs que sont la faible stabilité chromatique des produits dentaires finis (après réticulation) et de la cinétique de réticulation insuffisante lorsque des matériaux dentaires très épais (plusieurs millimètres d'épaisseur) sont préparés.

Selon un mode très avantageux, le choix du photosensibilisateur **(E)** et du photoamorceur cationique **(D)** se faisant de manière à ce qu'ils soient constitués du même anion, de préférence l'anion est choisi parmi les borates de formules suivantes : [B(C₆H₃(CF₃)₂)₄]⁻ et LB(C₆F₅)₄]⁻.

Comme mode de réalisation particulièrement préféré, le photosensibilisateur **(E),** éventuellement en association avec au moins une camphorquinone, une phénanthrènequinone et/ou un anthracène substitué est choisi parmi les composés de formule:

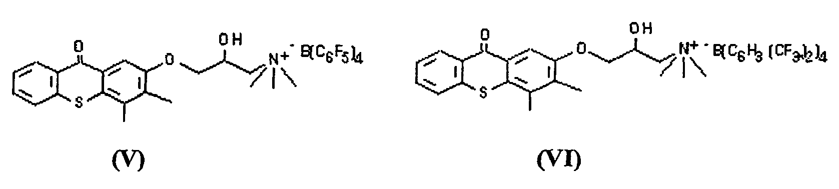

Parmi les composés **(A)** réactifs par voie cationique utiles selon l'invention on peut citer les monomères et/ou (co)polymères comprenant :
les époxy, les vinyles éthers, les oxétanes, les spiroorthocarbonates, les spiro-orthoesters et leurs associations.

Selon un mode de réalisation préféré, le composé **(A)** réactif par voie cationique est constitué par au moins un oligomère ou polymère silicone **(A-1)** réticulable et/ou polymérisable, liquide à température ambiante ou thermofusible à température inférieure à 100 °C, et comprenant :
a) au moins un motif de formule suivante : 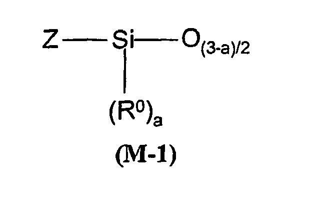 formule dans laquelle :
   - a=0, 1 ou 2,
   - R⁰, identique ou différent, représente un radical alkyle, cycloalkyle, aryle, vinyle, hydrogéno, alcoxy, de préférence un alkyle inférieur en C1-C6, et
   - Z, identique ou différent, est un substituant organique comportant au moins une fonction réactive oxirane, alcénylether, oxétane, dioxolane et/ou carbonate, et
b) au moins deux atomes de silicium.

Le motif **(M-1)** comprend préférentiellement des substituants Z choisis parmi le groupe constitué par les radicaux suivants : 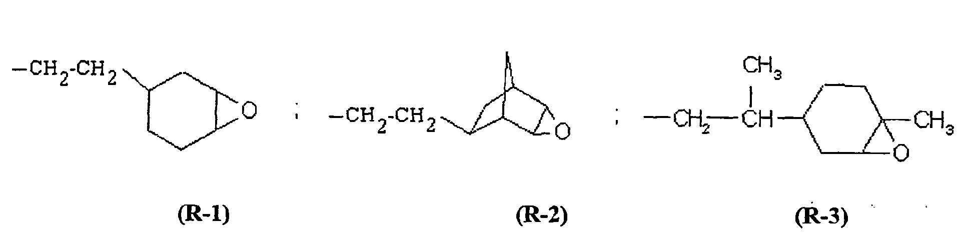 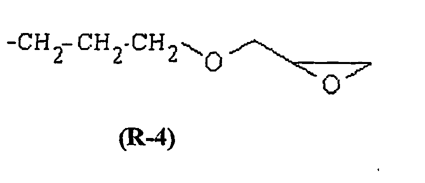 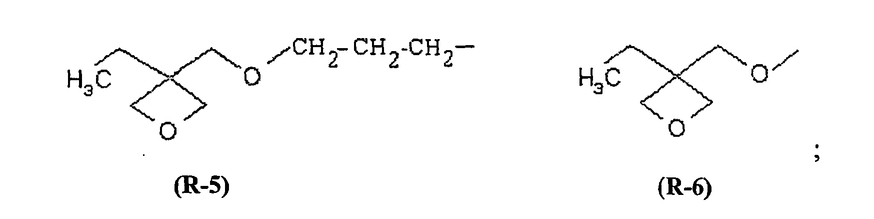 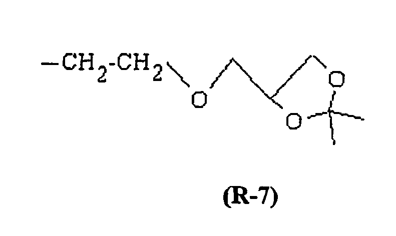 **(R-8)** -(CH₂)₃-O-CH=CH₂ ;

**(R-9)** -(CH₂)₃-O-CH=CH- R"

- avec R" représentant un radical alkyle linéaire ou ramifié en C₁-C₆.

Selon une deuxième variante avantageuse de la présente invention, le polymère ou oligomère silicone **(A-1)** est constitué par au moins un silicone dont la formule moyenne correspond à l'une des formules choisie parmi le groupe constitué par les formules **(S-1)** à **(S-92)** décrites ci-dessous :
**a)** 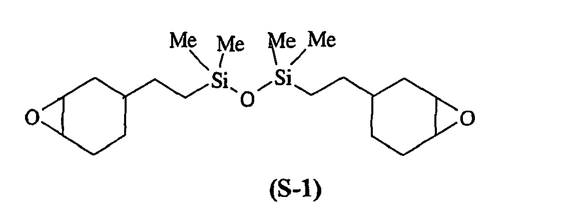
**b)** 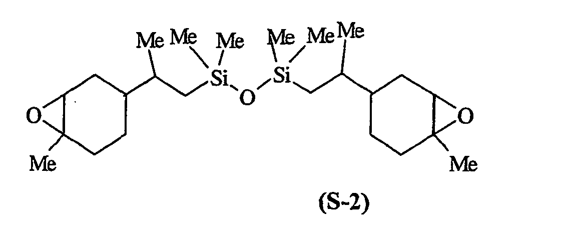
**c)** 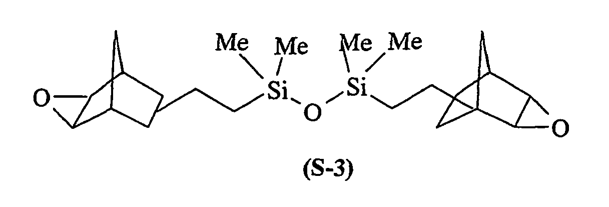
**d)** 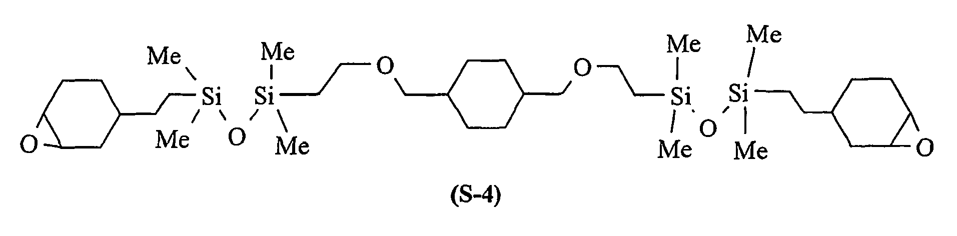
**e)** 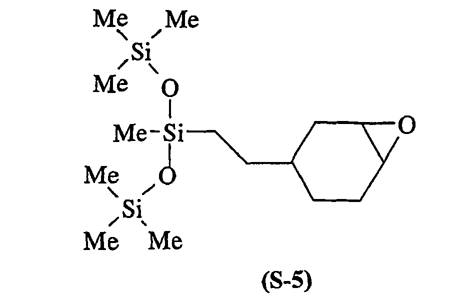
**f)** 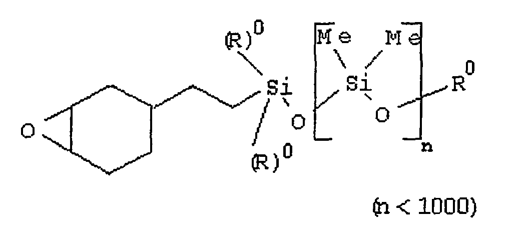
**g)** 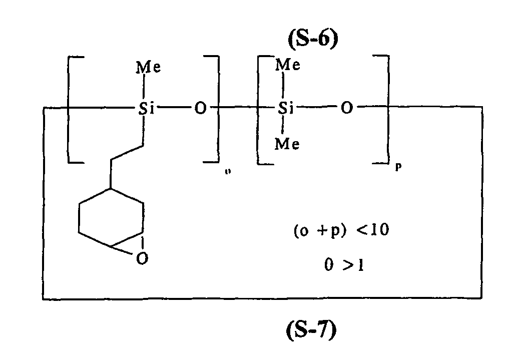
**h)** 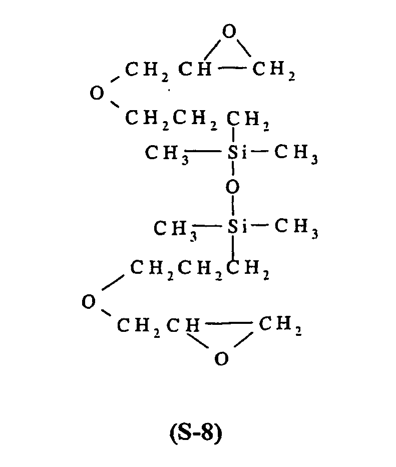
**i)** 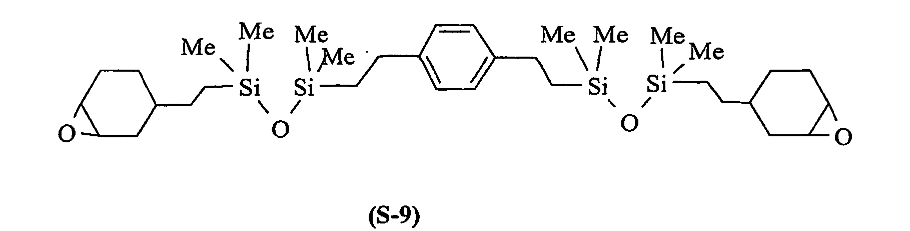
**j)** 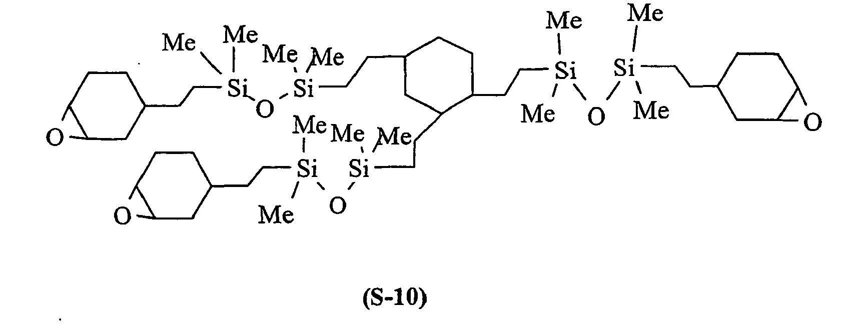
**k)** 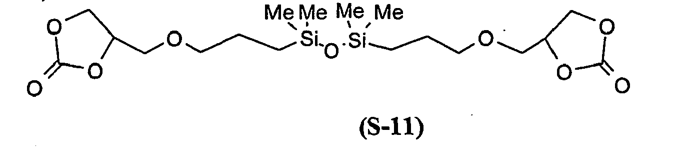
**l)** 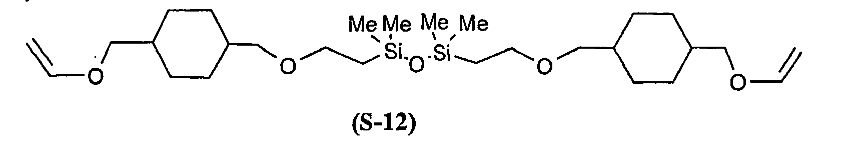
**m)** 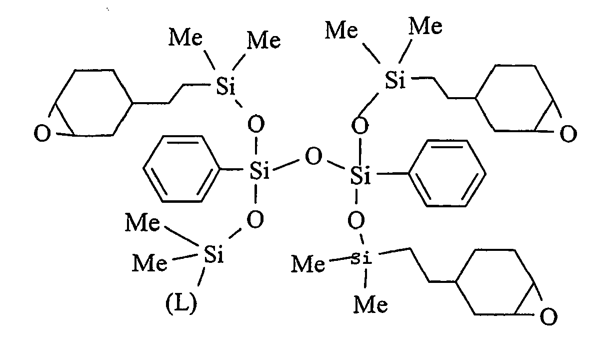 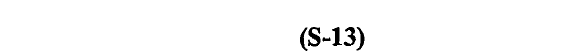 avec L= H ; OH ; Me ; Phényle ; alkyle en C1-C12 ; cycloalkyle C1-C6 ; ou les groupements : 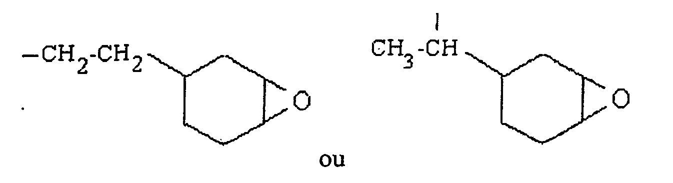
**n)** 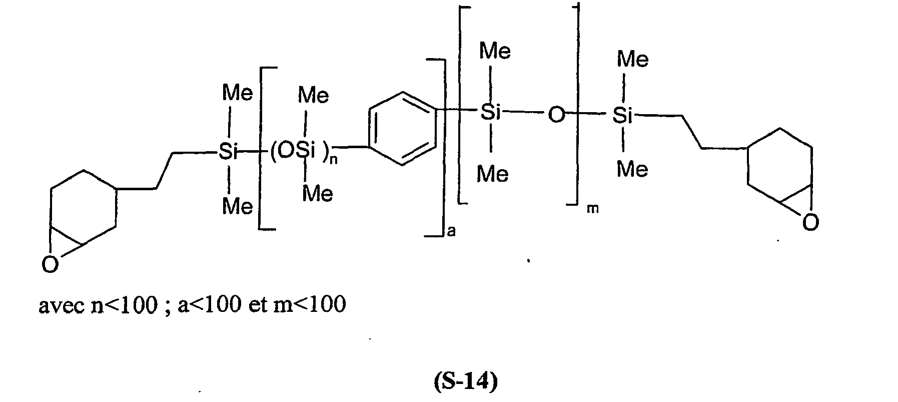
**o)** 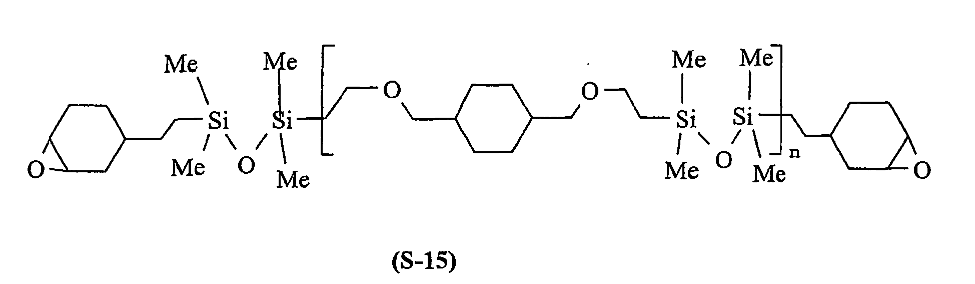
avec n<100
formules dans lesquelles R° ou R₀ , identiques ou différents, représente un radical alkyle, cycloalkyle, aryle, de préférence un alkyle inférieure en C1-C6. 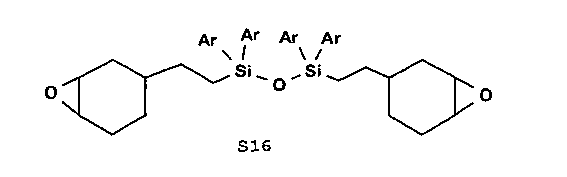 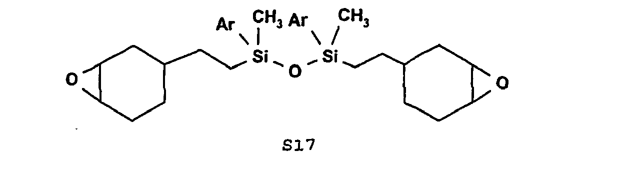 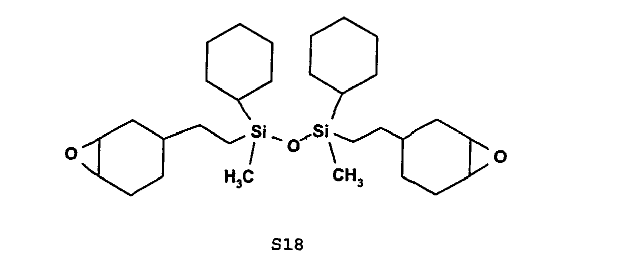 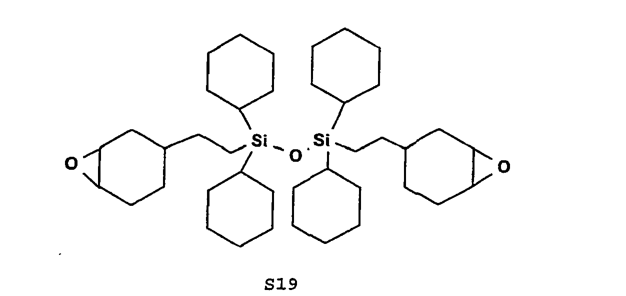 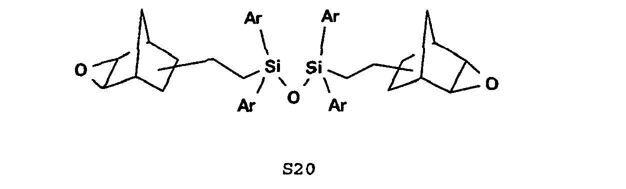 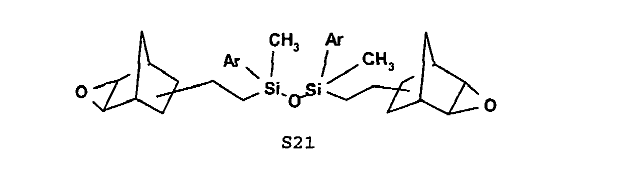 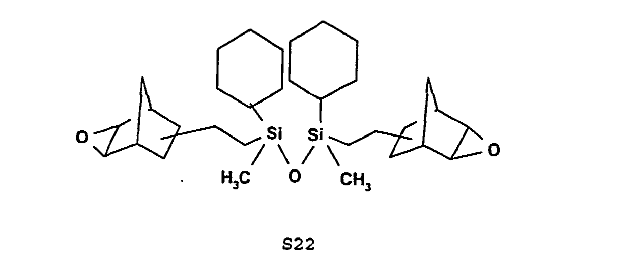 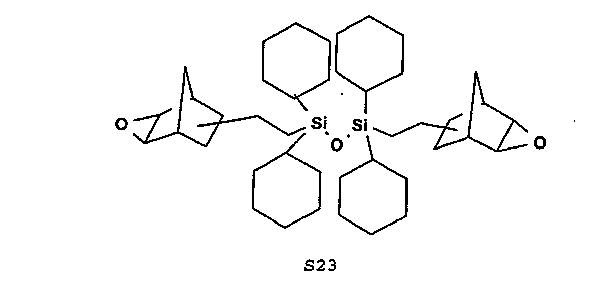 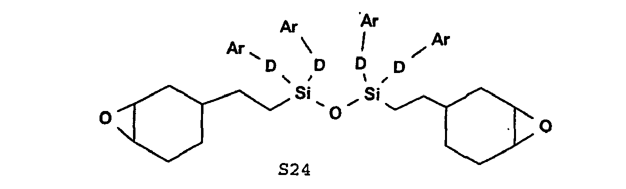 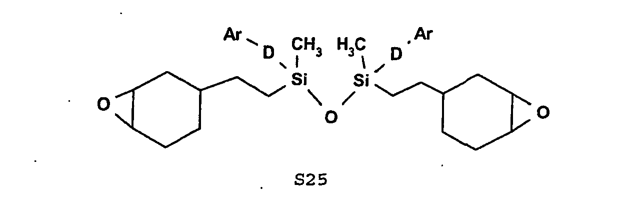 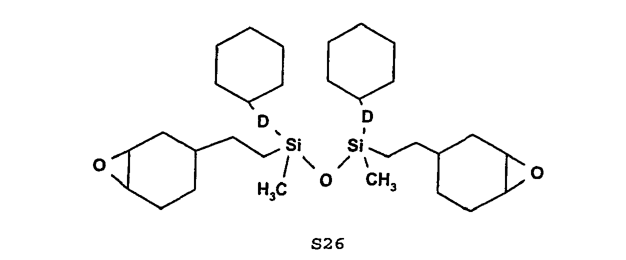 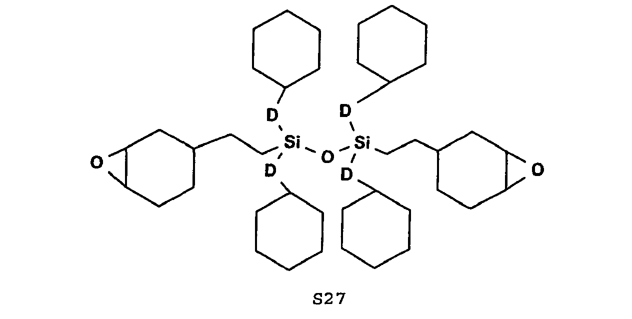 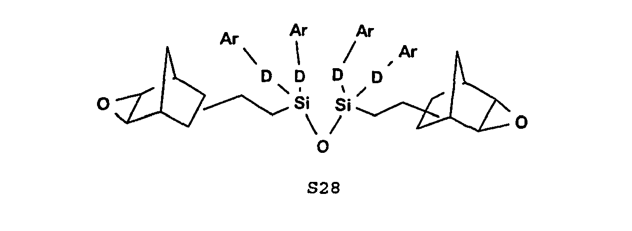 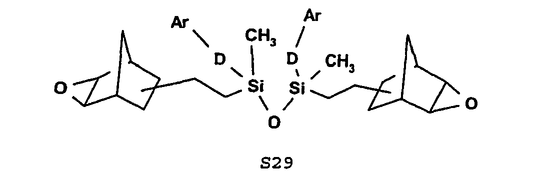 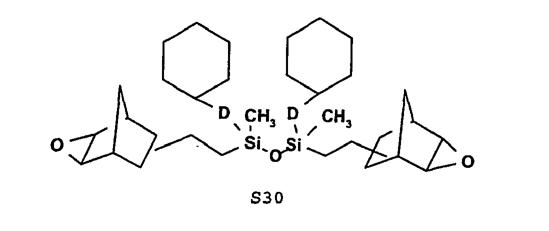 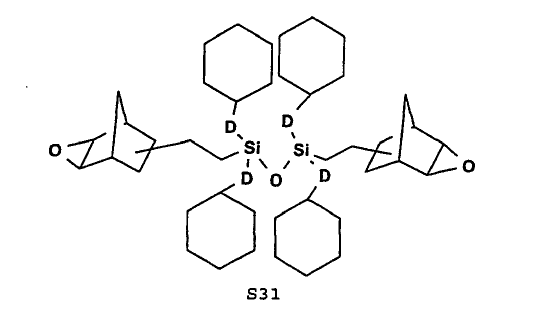 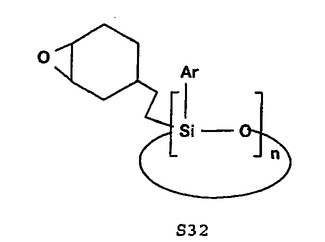 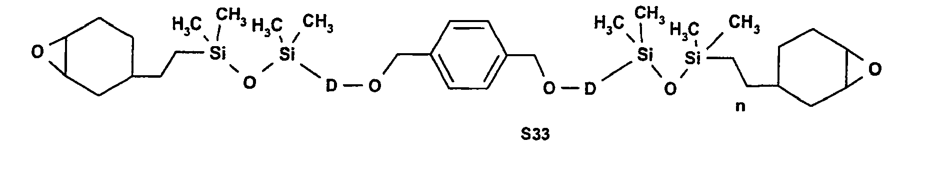 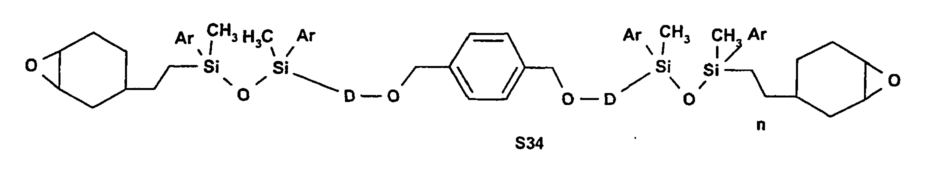 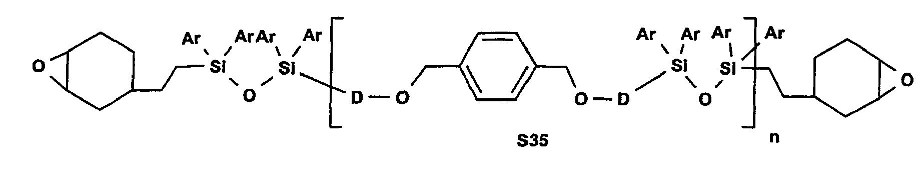 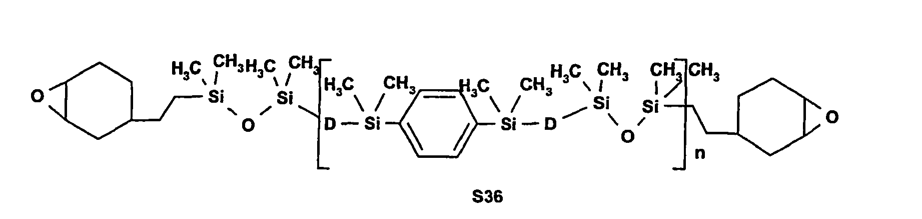 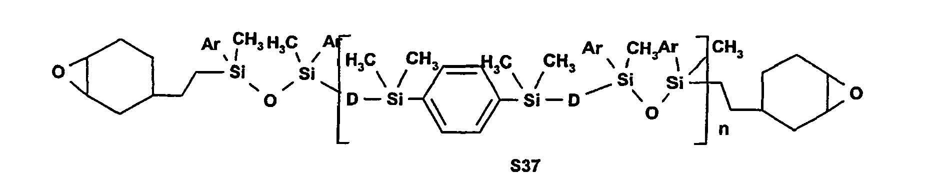 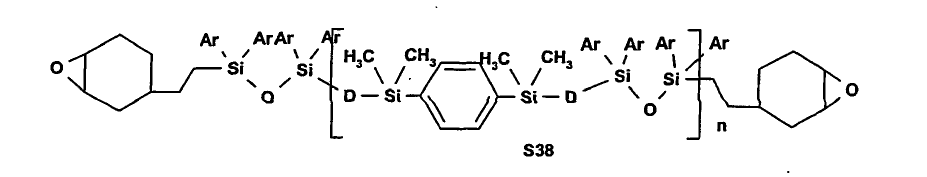 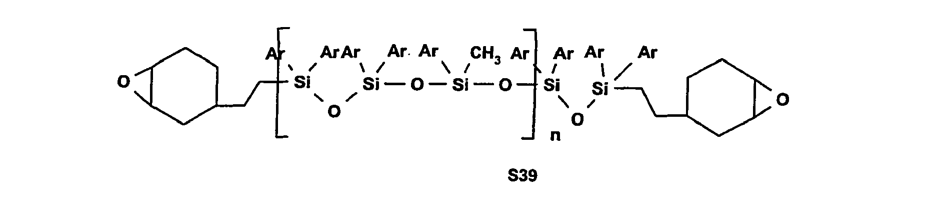 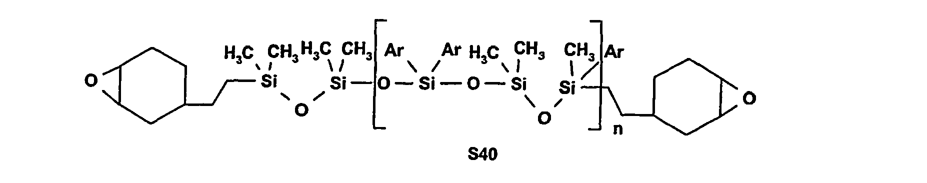 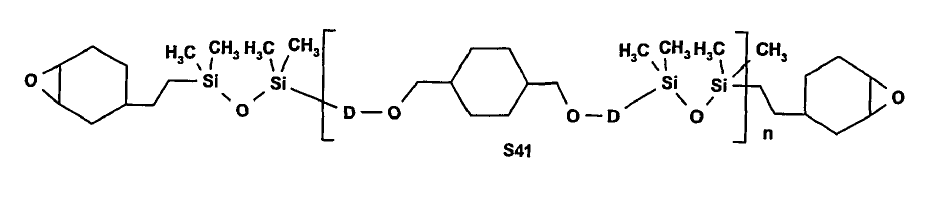 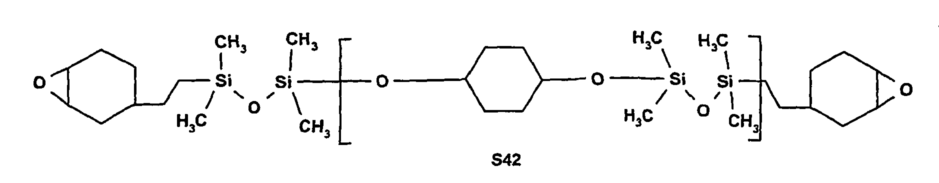 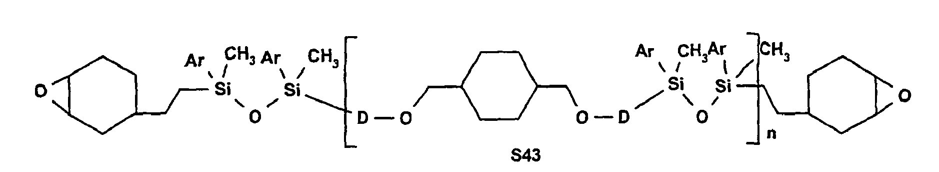 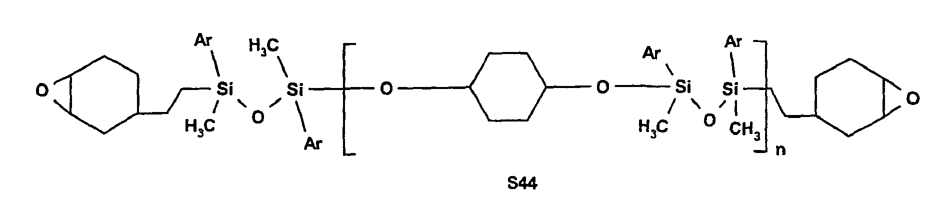 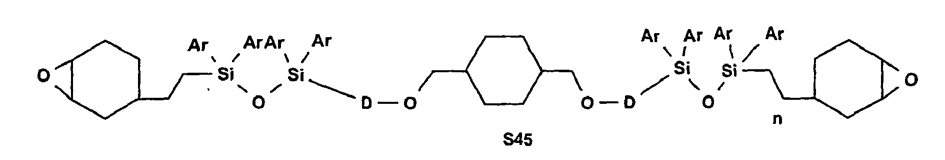 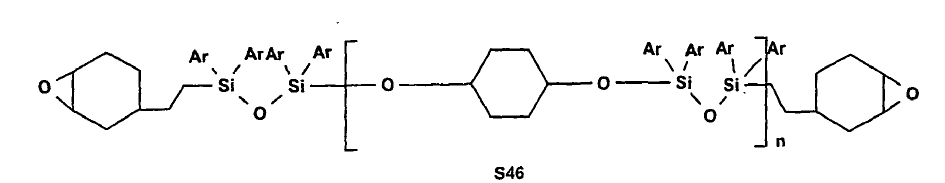 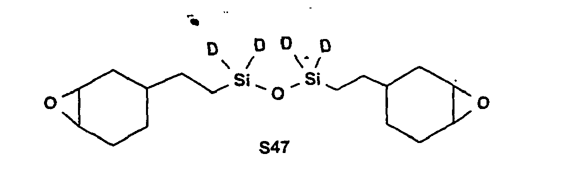 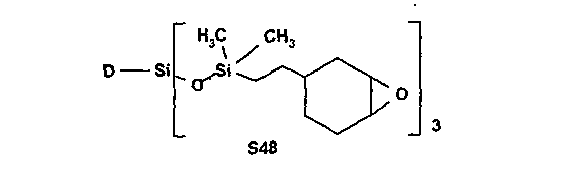 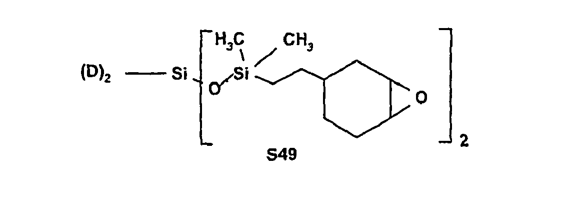 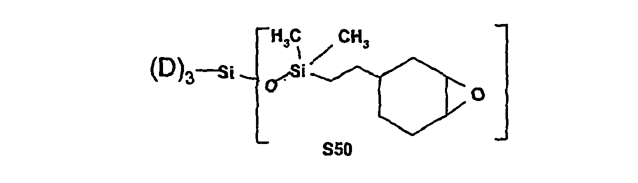 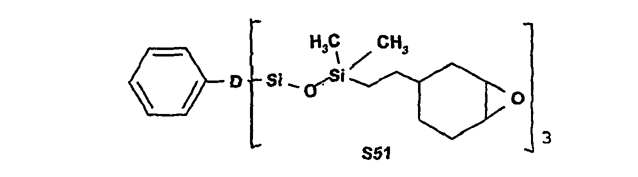 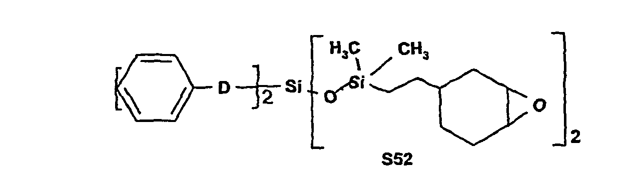 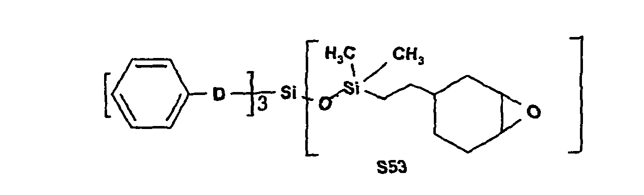 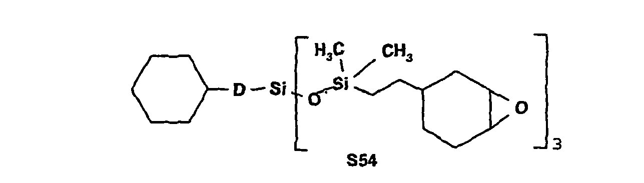 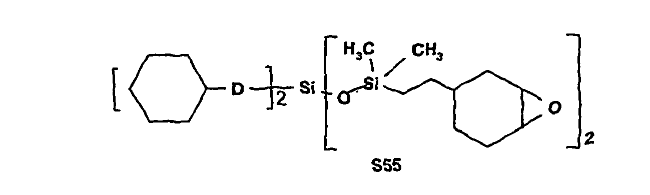 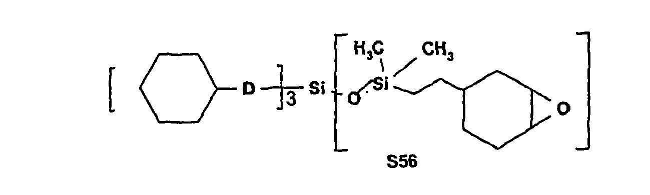 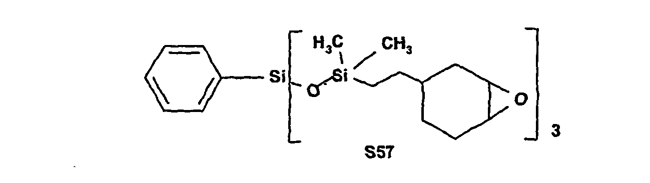 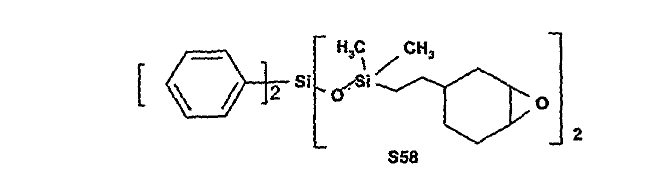 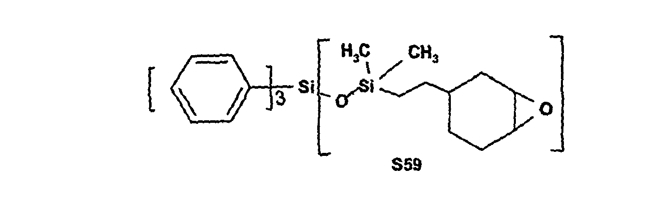 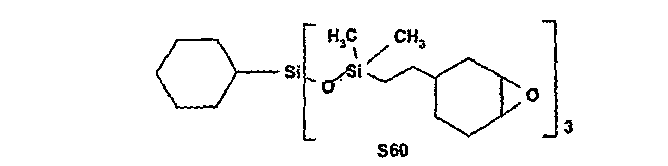 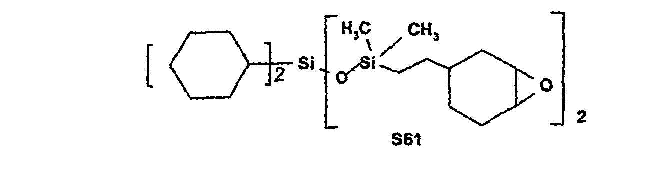 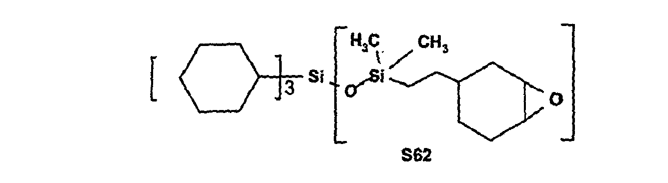 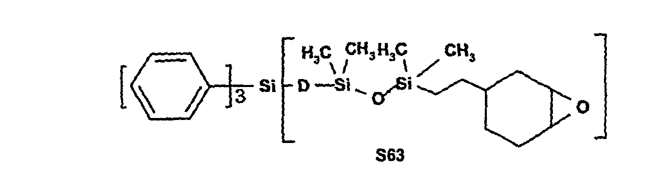 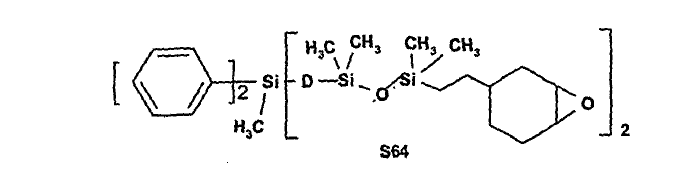 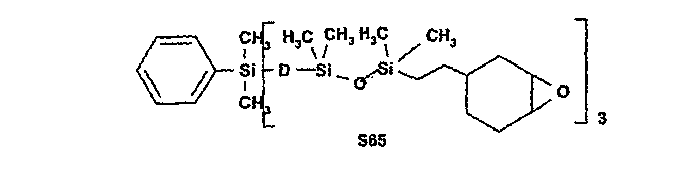 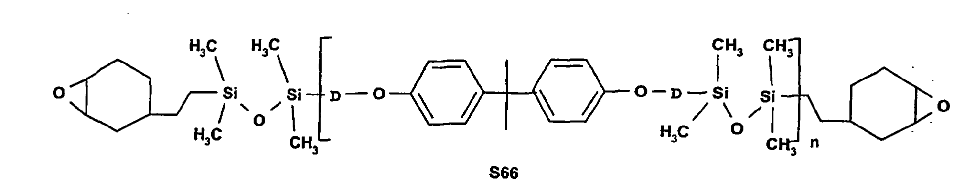 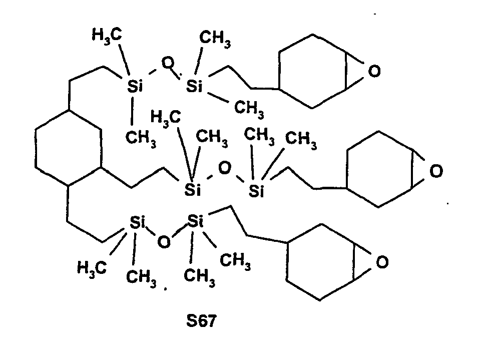 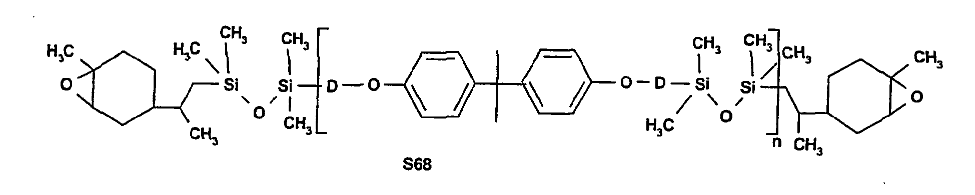 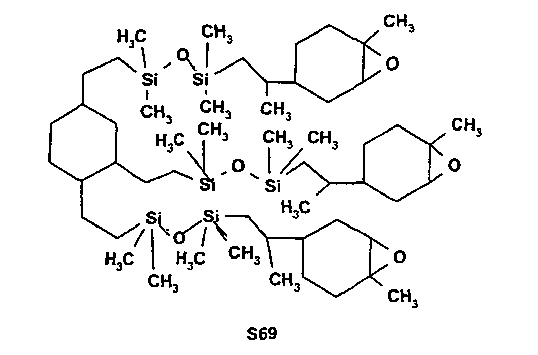 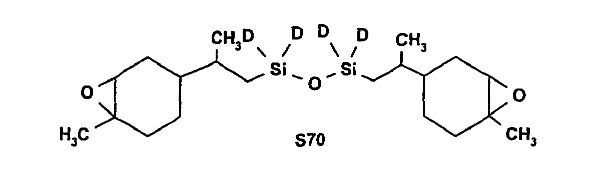 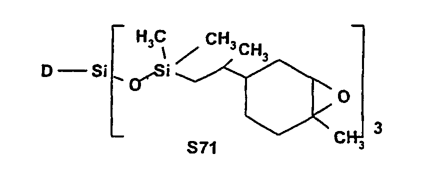 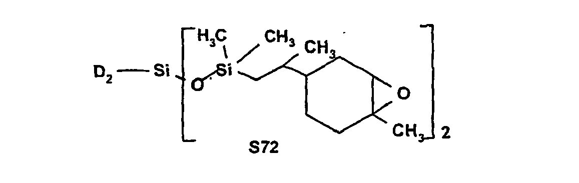 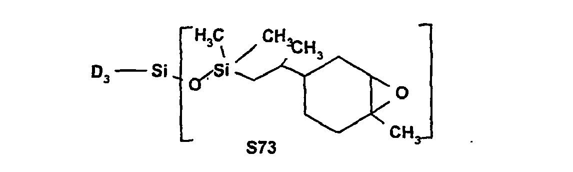 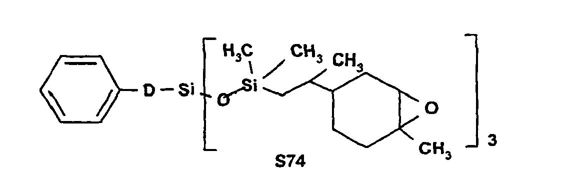 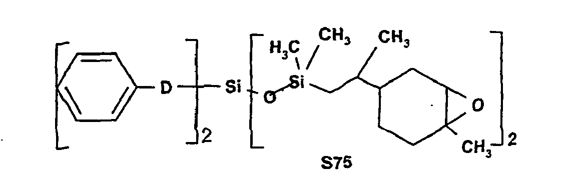 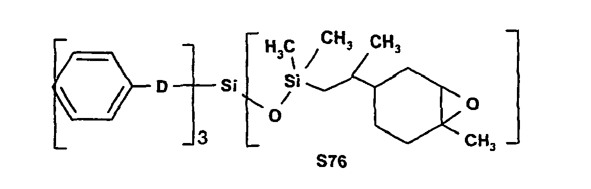 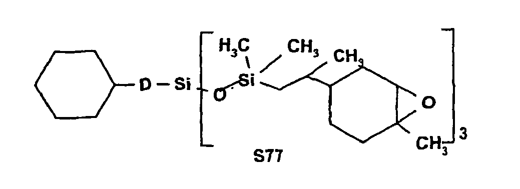 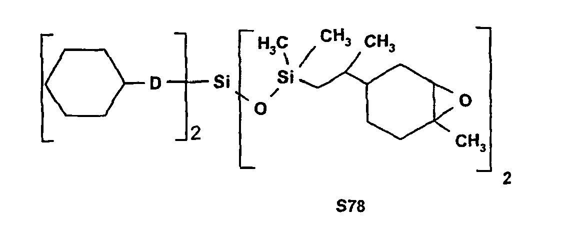 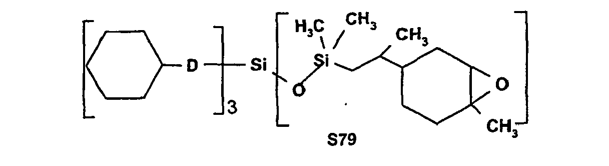 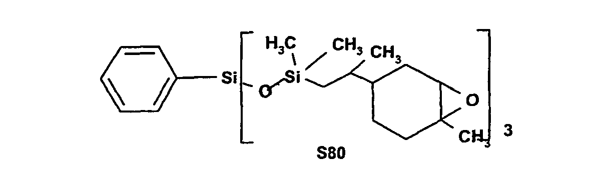 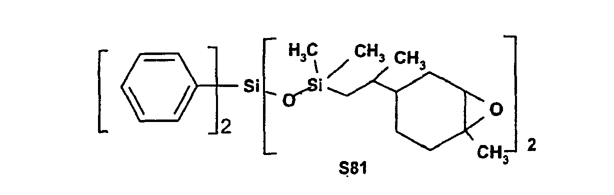 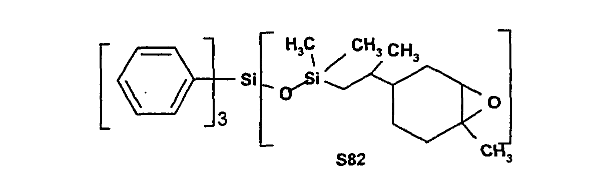 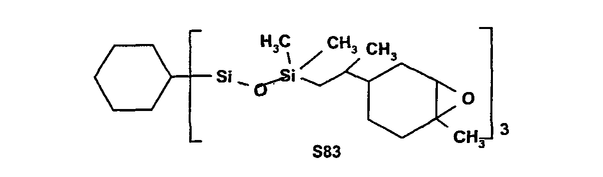 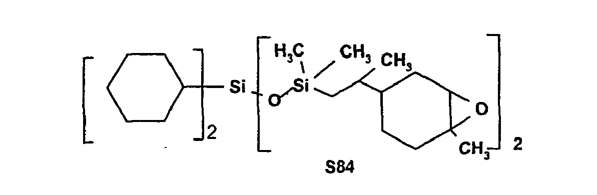 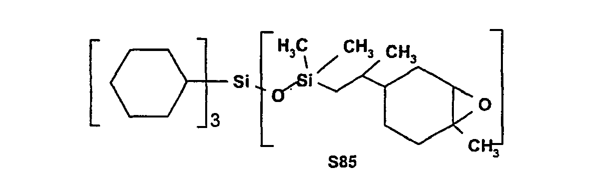 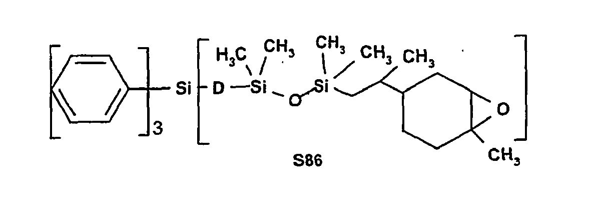 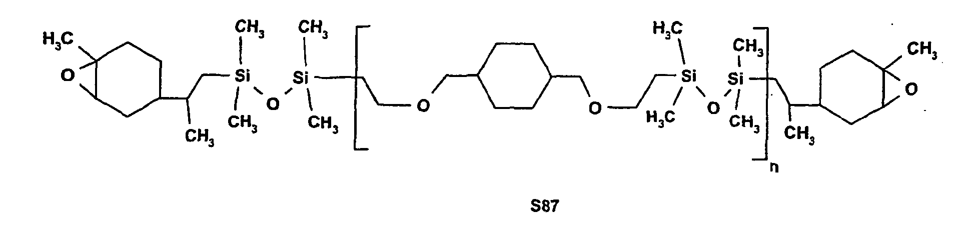 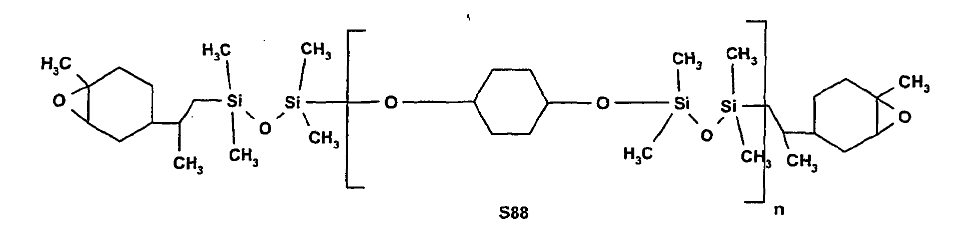 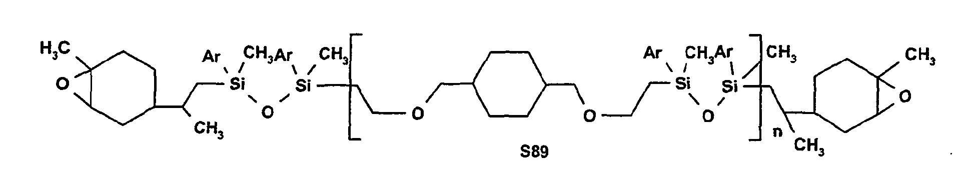 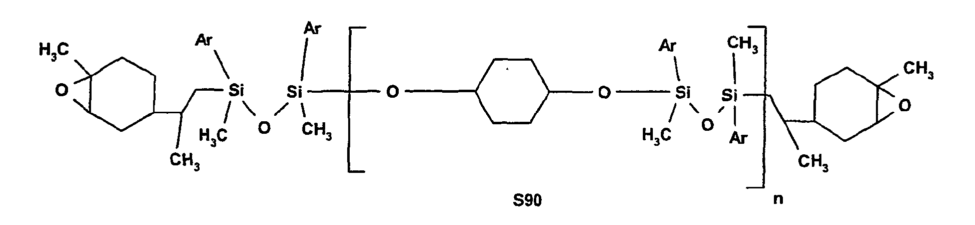 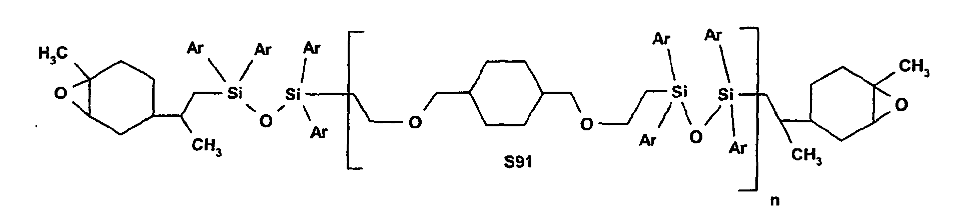 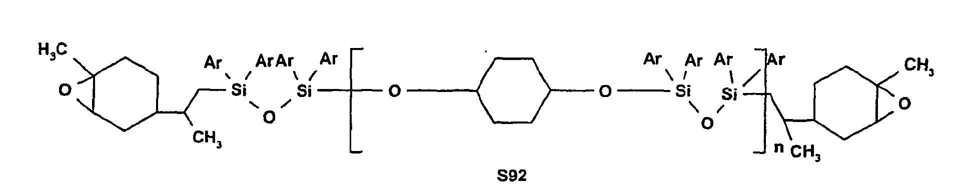 formules dans lesquelles le groupement D est un alkyle en C1-C12 linéaires ou ramifiés et n est un nombre entier compris entre 1 et 20 (bornes de l'intervalle incluses), avec Ar = groupe aryle.

Selon un mode de réalisation préféré, le composé **(A)** réactif par voie cationique est un silane **(G-3)** de formule : 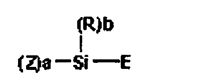 formule dans laquelle :
**E= -CH₂- ; -CH = ;** 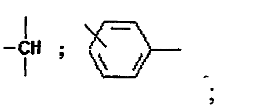
- R, identique ou différent, représente un radical alkyle, cycloalkyle, aryle, vinyle, hydrogéno, alcoxy, de préférence un alkyle inférieur en C1-C6,
- Z, identique ou différent, est un substituant organique comportant au moins une fonction oxirane, alcénylether, oxétane et/ou carbonate, et
- a+b=3.

Selon un mode de réalisation préféré, le silane **(G-3)** est choisi parmi le groupe constitué par les molécules **(S-93)** à **(S-95) :**

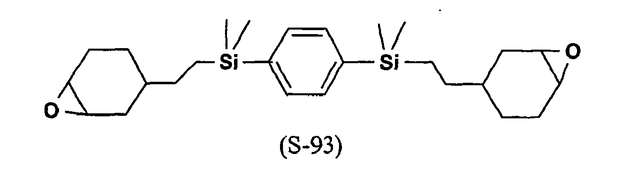

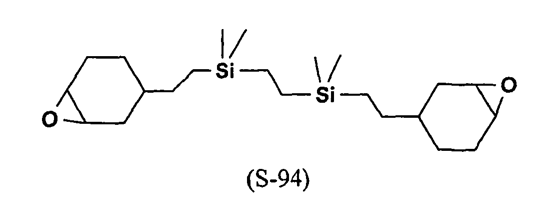

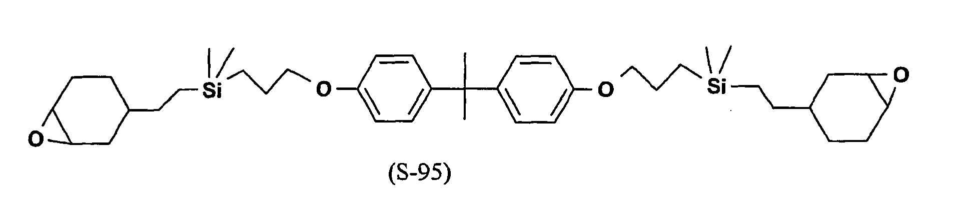

Selon un autre mode de réalisation préférentiel, le composé **(A)** réactif par voie cationique **(G)** est un composé organique **(G-4)** choisi parmi le groupe constitué par les molécules **(S-96)** à **(S-104) :** 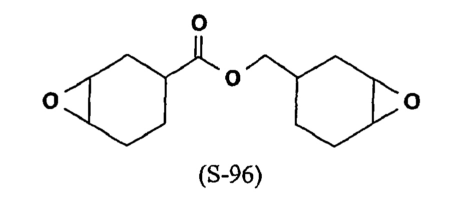 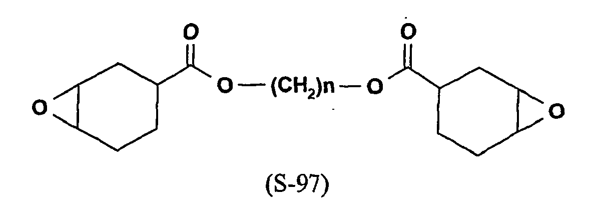 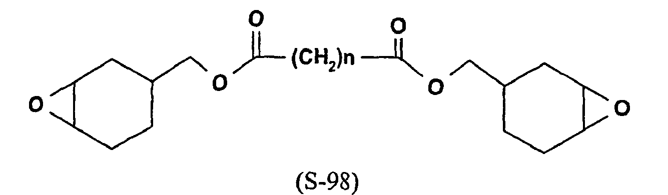 formules dans lesquelles : n est un nombre entier compris entre 1 et 10 (bornes incluses). 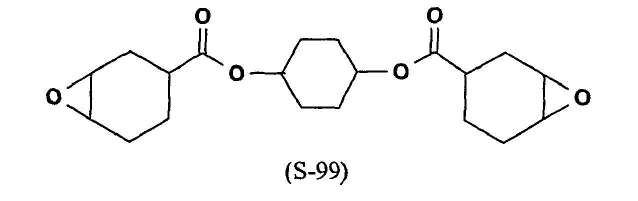 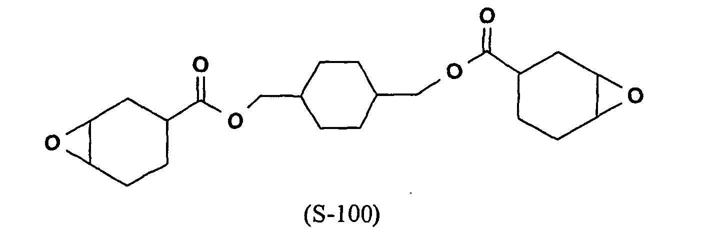 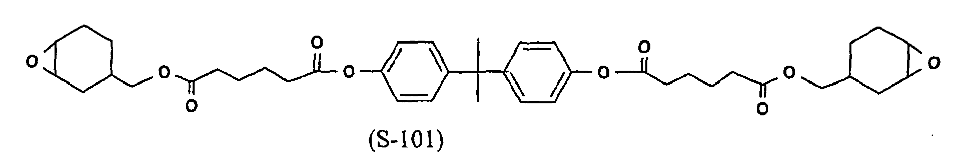 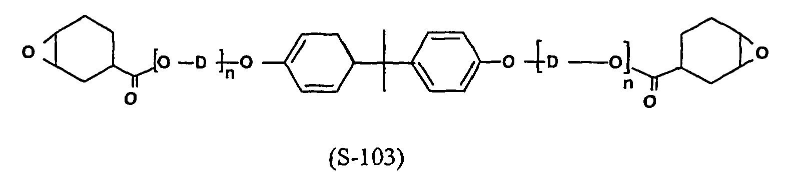 avec n<100 et D= alkyle en C₁-C₁₂ linéaires ou ramifiés.

Parmi les molécules de type **(S-103)** on peut choisir la résine UVR6150^{®} commercialisée par la société DOW-CHEMICAL ; et 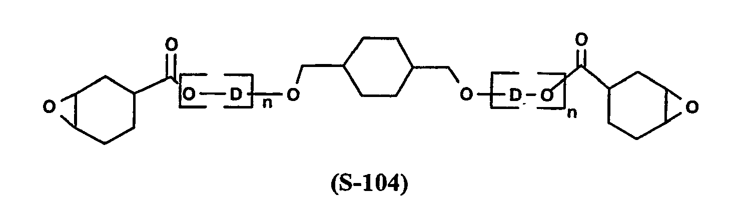 avec n<100 et le groupement D= alkyle en C1-C12 linéaires ou ramifiés.

Pour les résines de type **(S-104),** celle où n=0 est particulièrement adaptée à l'invention.

Selon une autre variante avantageuse de la présente invention, le composé **(A)** réactif par voie cationique est associé à une résine époxy organique ou oxétane représentant moins de 80 % en masse de la fraction le polymère ou oligomère silicone (A-1). Parmi les résines organiques fonctionnelles choisies on préférera celles dont le pourcentage massique de fonction réactive est inférieur à 20% et de préférence inférieur à 15%. On diminuera d'autant le retrait volumique lors de la polymérisation.

On choisira de préférence les résines de formule **(R-1)** et **(R-2)** 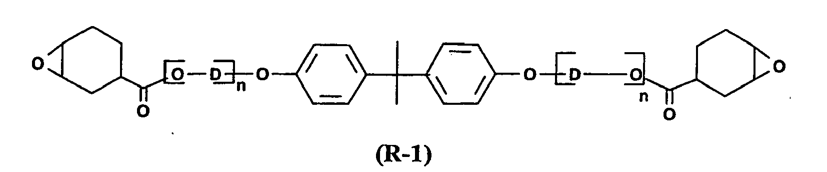 avec n<100 et D= alkyle en C1-C12 linéaires ou ramifiés.

Parmi les résines de type **(R-1)** on peut choisir la résine UVR6150 commercialisée par la société DOW-CHEMICAL. 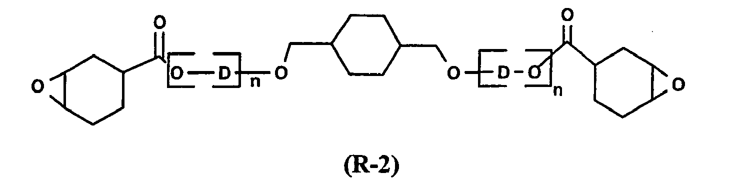 Avec n<100 et D= alkyle en C1-C12 linéaires ou ramifiés.

Parmi les résines de type **(R-2)** on peut choisir la résine où n=0.

Différents types de charge dentaire **(B)** sont utilisables pour préparer les compositions selon l'invention. Les charges sont choisies en fonction de l'utilisation finale de la composition dentaire : celles-ci affectent d'importantes propriétés telles que l'apparence, la pénétration du rayonnement U.V., ainsi que les propriétés mécaniques et physiques du matériau obtenu après réticulation et/ou polymérisation de la composition dentaire.

Comme charge de renforcement, on peut utiliser des charges de silice de pyrogénation traitée ou non, des charges de silice amorphe, du quartz, des verres ou des charges non vitreuses à base d'oxydes de silicium par exemple du type de celles décrites dans le brevet US-6,297,181 (sans baryum), de zirconium, de baryum, de calcium, de fluor, d'aluminium, de titane, de zinc, des borosilicates, des aluminosilicates, du talc, des sphérosil, du trifluorure d'yterbium, des charges à base de polymères sous forme de poudre broyée tel que des polyméthacrylates de méthyle inertes ou fonctionnalisés, des polyépoxydes ou des polycarbonates, des whiskers de céramiques (Si-C, Si-O-C, Si-N, Si-N-C, Si-N-C-O), des fibres de verre.

A titre d'exemple, on citera :
- des charges inertes à base de polyméthacrylate de méthyle LUXASELF^{®} commercialisé par la société UGL utilisables dans le domaine dentaire et pigmentées en rose,
- des charges de silice de combustion traitée hexaméthyldisilazane ou polydiméthylsiloxane de surface spécifique 200 m /g,
- des charges de silice de combustion non traitée (AEROSIL-AE200^{®} ou OX50^{®} commercialisées par la société DEGUSSA), et
- des verres à base d'oxydes de silicium, d'oxyde de baryum et/ou d'oxyde de strontium.

Selon un mode de réalisation préféré, la charge dentaire **(B)** est un verre minéral ou une silice de combustion.

Suivant une caractéristique avantageuse de l'invention, la charge dentaire **(B)** représente jusqu'à 85 % en poids, de préférence entre 50 et 85 % en poids, encore plus préférentiellement entre 60 et 85 % en poids, par rapport au poids total de la composition dentaire.

Conformément à l'invention, le dispersant **(C)** est sélectionné dans le groupe comprenant: les copolymères polyuréthanne/acrylate éventuellement salifiés par un alkylammonium, les copolymères acryliques éventuellement salifiés par un alkylammonium, les monodiesters d'acides carboxyliques, les polyesters, les polyéthers, les polyuréthannes, les polyuréthannes modifiés, les polyol-polyacrylates, leurs copolymères ou leurs mélanges. Les dispersants commercialisés sous la marque DISPERBYK^{®} (de la société Byk) ou SOLSPERSE^{®} (de la société Avecia) conviennent particulièrement à l'invention. On peut citer en particulier et à titre d'exemples, les produits commerciaux : Disperbyk^{®} 164, Disperbyk^{®} 161, Disperbyk^{®}166, Disperbyk^{®}2070, Disperbyk^{®} 9075, Disperbyk^{®} 9076. On peut citer aussi les dispersants cités dans les brevets suivants :
- le brevet US-5,882,393 décrivant des dispersants à base de polyuréthannes/imidazoles-acrylates ou époxydes ;
- le brevet US-5,425,900 décrivant des dispersants à base de polyuréthannes;
- le brevet US-4,795,796 décrivant des dispersants à base de polyuréthannes/polyoxyalkylène-glycolmonoalkyléther;
- la demande de brevet WO-A-99/56864 décrivant des dispersants à base de polyuréthannes/poly(oxyalkylène-carbonyle): dérivés de ε-caprolactone et de δ-valérolactone ; et
- le brevet EP-0 403 197 décrivant des dispersants de type polyol-polyacrylate greffé comprenant un copolymère statistique polyuréthanne/polyvinylique/polyacrylate et un polyoxyalkylène polyéther.

Quantitativement parlant, le dispersant **(C)** est présent à raison de 50 ppm à 1 %, de préférence 100 ppm à 5000 ppm.

De préférence, l'indice d'amine du dispersant **(C)** est inférieur ou égal à 60, et plus préférablement encore compris entre 0,1 et 50 mg de potasse par gramme de dispersant **(C).**

Avantageusement, l'indice d'acide du dispersant est inférieur ou égal à 200, de préférence inférieur ou égal à 100, et plus préférablement compris entre 1 et 60 mg de potasse par gramme de dispersant.

Les photoamorceurs cationiques **(D)** sont choisis parmi les borates d'onium (pris à eux seuls ou en mélange entre eux) d'un élément des groupes 15 à 17 de la classification périodique [Chem. & Eng. News, vol.63, N° 5, 26 du 4 février 1985] ou d'un complexe organométallique d'un élément des groupes 4 à 10 de la classification périodique [même référence].

Selon un mode préférentiel le photoamorceur cationique **(D)** est de type borate et est choisi parmi ceux dont :
a) l'entité cationique du borate est sélectionnée parmi :
   (1) les sels d'onium de formule:

      [(R¹)ₙ - A - (R²)ₘ]⁺ **(VII)**

      formule dans laquelle :
      - A représente un élément des groupes 15 à 17 tel que par exemple : I, S, Se, P ou N,
      - R¹ représente un radical aryle carbocyclique ou hétérocyclique en C6-C20, ledit radical hétérocyclique pouvant contenir comme hétéroéléments de l'azote ou du soufre,
      - R² représente R¹ ou un radical alkyle ou alkényle linéaire ou ramifié en C1-C30 ; - lesdits radicaux R¹ et R² étant éventuellement substitués par un groupement alcoxy en C1-C25, alkyle en C1-C25, nitro, chloro, bromo, cyano, carboxy, ester ou mercapto,
      - m et n sont des nombres entiers, avec n + m = v + 1, v étant la valence de l'élément A,
   (2) les sels d'oxoisothiochromanium en l'occurrence ceux décrits dans la demande de brevet WO 90/11303, notamment le sel de sulfonium du 2-éthyl-4-oxoisothiochromanium ou de 2-dodécyl-4-oxoisothio-chromanium et les sels d'oxoisothiochromanium de formule structurale V : 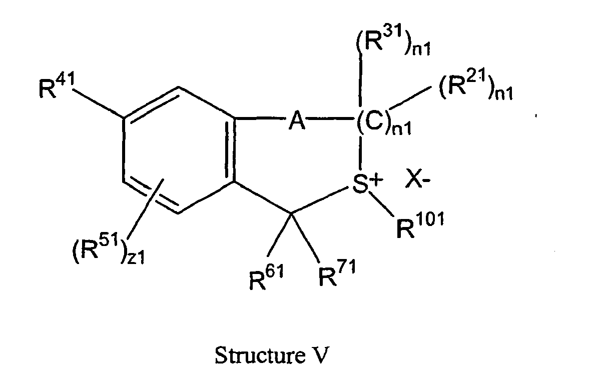 Formule dans laquelle :
      A représente : 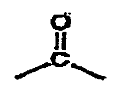
      n1 = un entier entre 1 et 3 ;
      zl = un entier entre 0 et 3 ;
      X représente un groupe de fonnule M¹Y¹ᵣ₁) (1) ou de fonnule Q¹(2), où dans M¹Y¹ᵣ₁; (1) : M¹ = Sb, As, P, B ou Cl, Y¹ représente un halogène (de préférence F ou Cl) ou O et où r1 est un entier entre 4 et 6, la formule Q¹(2) représente un acide sulfonique ,
      R⁸¹-SO₃ où R⁸¹ est un groupe alkyle ou aryle, ou un groupe alkyle ou aryle substitué par un halogène, de préférence F ou C1,
      R¹⁰¹ représente un groupe alkyle ou cycloalkyle, de préférence en C₁-C₂₀, ou un groupe aryle,
      R²¹ représente un hydrogène, un groupe alkyle, alcényle, cycloalcényle ou cycloalkyle, de préférence C₁-C₂₀, ou un groupe aryle, tous les R²¹ étant indépendants les uns des autres,
      R³¹ représente un hydrogène, un groupe alkyle, alcényle, cycloalcényle ou cycloalkyle, de préférence C₁-C₂₀, ou un groupe aryle, tous les R³¹ étant indépendants les uns des autres,
      R⁴¹ représente un hydrogène, un halogène, un groupe alcényle, par exemple vinyle, cycloalcényle, alkyle, ou cycloalkyle, de préférence en en C₁-C₂₀, un groupe alcoxy ou thioalcoxy, de préférence en en C₁-C₂₀, un groupe poly(alkylènoxyde) avec jusqu'à 10 unités alkylènoxydes terminé par un hydroxyle ou un alkyle (C₁-C₁₂), un groupe aryle, un groupe aryloxy ou thioaryloxy,
      R⁵¹ représente un halogène, un groupe alcényle, par exemple vinyle, cycloalcényle, alkyle, ou cycloalkyle, de préférence en en C₁-C₂₀, un groupe alcoxy ou thioalcoxy, de préférence en en C₁-C₂₀, un groupe poly(alkylènoxyde) avec jusqu'à 10 unités alkylènoxydes terminé par un hydroxyle ou un alkyle (C₁-C₁₂), un groupe aryle, un groupe aryloxy ou thioaryloxy,
      R⁶¹ représente un hydrogène, un groupe alkyle, alcényle, cycloalcényle ou cycloalkyle, de préférence en C₁-C₂₀, ou un groupe aryle,
      R⁷¹ représente un hydrogène, un groupe alkyle, alcényle, cycloalcényle ou cycloalkyle, de préférence en C₁-C₂₀, ou un groupe aryle; et
   (3) les sels organométalliques de formule suivante:

      (L₁L₂L₃M^{)+q} **(VIII)**

      formule dans laquelle :
      - M représente un métal du groupe 4 à 10, notamment du fer, manganèse, chrome, cobalt,
      - L1 représente 1 ligand lié au métal M par des électrons π, ligand choisi parmi les ligands η³-alkyl, η⁵- cyclopendadiènyl et η⁷-cycloheptratriènyl et les composés η⁶ - aromatiques choisis parmi les ligands η⁶-benzène éventuellement substitués et les composés ayant de 2 à 4 cycles condensés, chaque cycle étant capable de contribuer à la couche de valence du métal M par 3 à 8 électrons π,
      - L2 représente un ligand lié au métal M par des électrons π, ligand choisi parmi les ligands η⁷-cycloheptatriènyl et les composés η⁶-aromatiques choisis parmi les ligands η⁶- benzène éventuellement substitués et les composés ayant de 2 à 4 cycles condensés, chaque cycle étant capable de contribuer à la couche de valence du métal M par 6 ou 7 électrons π,
      - L3 représente de 0 à 3 ligands identiques ou différents liés au métal M par des électrons σ, ligand(s) choisi(s) parmi CO et N02+ ; la charge électronique totale q du complexe à laquelle contribuent L1, L2 et L3 et la charge ionique du métal M étant positive et égale à 1 ou 2 ; et
b) ceux dont l'entité anionique borate a pour formule :

   [BXₐ R_{b}]⁻ **(I)**

   formule dans laquelle :
   - a et b sont des nombres entiers allant pour a de 0 à 3 et pour b de 1 à 4 avec a + b = 4,
   - les symboles X représentent :
      * un atome d'halogène (chlore, fluor) avec a = 0 à 3, ou
      * une fonction OH avec a = 0 à 2,
   - les symboles R sont identiques ou différents et représentent :
      - un radical phényle substitué par au moins un groupement électroattracteur tel que par exemple OCF₃, CF₃, NO₂, CN, et/ou par au moins 2 atomes d'halogène (fluor tout particulièrement), et ce lorsque l'entité cationique est un onium d'un élément des groupes 15 à 17,
      - un radical phényle substitué par au moins un élément ou un groupement électroattracteur notamment atome d'halogène (fluor tout particulièrement), CF₃, OCF₃, NO₂, CN, et ce lorsque l'entité cationique est un complexe organométallique d'un élément des groupes 4 à 10, ou
      - un radical aryle contenant au moins deux noyaux aromatiques tel que par exemple biphényle, naphtyle, éventuellement substitué par au moins un élément ou un groupement électroattracteur, notamment un atome d'halogène dont le fluor en particulier, OCF₃, CF₃, NO₂, CN, quelle que soit l'entité cationique.

Selon un mode de réalisation préféré, le photoamorceur cationique **(D)** est un sel d'iodonium.

Sans que cela ne soit limitatif, sont données ci-après plus de précisions quant aux sous classes de borate d'onium et de borate de sels organométalliques plus particulièrement préférés dans le cadre de l'utilisation conforme à l'invention.

Selon une première variante préférée de l'invention, les espèces de l'entité anionique borate qui conviennent tout particulièrement sont les suivantes :

| | | | |
|---|---|---|---|
| **1':** | [B(C₆F₅)₄]⁻ | **5':** | [B(C₆H₃(CF₃)₂)₄]⁻ |
| **2':** | [(C₆F₅)₂BF₂]⁻ | **6':** | [B (C₆H₃F₂)₄]⁻ |
| **3':** | [B(C₆H₄CF₃)₄]⁻ | **7':** | [C₆F₅BF₃]⁻ |
| **4':** | [B(C₆F₄OCF₃)₄]⁻. | | |

Selon une deuxième variante préférée de l'invention, les sels d'onium de formule **(VII)** utilisables sont décrits dans de nombreux documents notamment dans les brevets US-A-4 026 705, US-A-4 032 673, US-A-4 069 056, US-A-4 136 102, US-A-4 173 476. Parmi ceux-ci, on privilégiera tout particulièrement les cations suivants :
[(C₈H₁₇)-O- (C₆H₄)-I- C₆H₅)]⁺; [C₁₂H₂₅- (C₆H₄)-I-C₆H₅]⁺ ; [(C₈H₁₇-O- (C₆H₄))₂I]⁺
[(C₈H₁₇)-O- (C₆H₄)-I- C₆H₅)]⁺; [(C₆H₅)S-(C₆H₄)-O-C₈H₁₇]⁺;
[CH₃- C₆H₄-I- C₆H₄-CH(CH(CH₃)₂]⁺ ; [(C₁₂H₂₅-(C₆H₄)-I-(C₆H₄)-CH-(CH₃)₂]⁺
[(C₁₂H₂₅- C₆H₄)₂I]⁺ ; [(C₆H₅)₃ S]⁺; [CH₃-(C₆H₄)-I-(C₆H₄)-CH(CH₃)₂]⁺
(η5 - cyclopentadiènyle) (η6 - toluène) Fe⁺ ; (η5 - cyclopentadiènyle) (η6 - cumène) Fe⁺,
(η5 - cyclopentadiènyle) (η6-méthyl-1-naphtalène) Fe⁺;
[(C₆H₅)-S-C₆H₄-S-(C₆H₅)₂]⁺ ; [(CH₃-(C₆H₄)-I-(C₆H₄)-OC₂H₅]⁺;
[(CₙH₂ₙ₊₁- C₆H₄)₂I] ⁺ avec n= 1 à 18.

Selon une troisième variante préférée, les sels organométalliques (3) de formule **(VIII)** utilisables sont décrits dans les documents US-A-4 973 722, US-A-4 992 572, EP-A-203 829, EP-A-323 584 et EP-A-354 181. Les sels organométalliques plus volontiers retenus selon l'invention sont notamment :
. le (η⁵ - cyclopentadiènyle) (η⁶ - toluène) Fe⁺,
. le (η⁵ - cyclopentadiènyle) (η⁶ - méthyl-1-naphtalène) Fe⁺,
. le (η⁵ - cyclopentadiènyle) (η⁶ - cumène) Fe⁺,
. le bis (η⁶ - mesitylène) Fe⁺,
. le bis (η⁶ - benzène) Cr⁺

En accord avec ces trois variantes préférées, on peut citer, à titre d'exemples de photoamorceurs du type borates d'onium, les produits suivants :
**(P-16) :** [(C₈H₁₇)-O- C₆H₄-I- C₆H₅)]⁺, [B(C₆F⁵)₄];
**(P-17) :** [C₁₂H₂₅- C₆H₄-I-C₆H₅]⁺, [B(C₆F₅)₄]⁻ ;
**(P-18) :** [(C₈H₁₇-O- C₆H₄)₂I]⁺, [B(C₆F₅)₄]⁻;
**(P-19) :** [(C₈H₁₇)-O- C₆H₄-I- C₆H₅)]⁺, [B(C₆F₅)₄]⁻;
**(P-20) :** [(C₆H₅)₂S- C₆H₄-O-C₈H₁₇]⁺, [B(C₆H₄CF₃)₄]⁻;
**(P-21):** [(C₁₂H₂₅- C₆H₄)₂I]⁺ , [B (C₆F₅)₄]⁻;
**(P-22) :** [CH₃- C₆H₄-I- C₆H₄-CH(CH₃)₂]⁺ , [B(C₆F₅)₄]⁻;
**(P-23) :** (η5 - cyclopentadiènyle) (η6 - toluène) Fe⁺, [B(C₆F₅)₄] ;
**(P-24) :** (η5 - cyclopentadiènyle) (η6 - methyl-1-naphtalène) Fe⁺, [B(C₆F₅)₄]⁻ ;
**(P-25) :** (η5 - cyclopentadiènyle) (η6 - cumène) Fe⁺, [B(C₆F₅)₄]⁻;
**(P-26) :** [C₁₂H₂₅- C₆H₄)₂I]⁺, [B(C₆H₃(CF₃)₂]⁻ ;
**(P-27) :** [CH₃- C₆H₄-I- C₆H₄-CH₂CH(CH₃)₂]⁺, [B(C₆F₅)₄]⁻ ;
**(P-28) :** [CH₃- C₆H₄-I- C₆H₄-CH₂CH(CH₃)₂]⁺ , [B(C₆H₃(CF₃) ₂)₄]⁻.; et
**(P-29) :** [CH₃- C₆H₄-I- C₆H₄-CH(CH₃)₂]⁺, [B(C₆H₃(CF₃) ₂) ₄]⁻.

Comme autre référence pour définir les borates d'onium (1) et (2) et les borates de sels organométalliques (3), on peut citer l'ensemble du contenu des demandes de brevet EP 0 562 897 et 0 562 922.

Comme autre exemple de sel d'onium utilisable comme photoamorceur, on peut citer ceux divulgués dans les brevets américains US 4 138 255 et US 4 310 469.

On peut également utiliser d'autres photoamorceurs cationiques, par exemple:
- les sels d'iodonium hexafluorophosphate ou hexafluoro-antimonate, tels que :
   - [CH₃-[(C₆H₄)-I-[(C₆H₄)-CH(CH₃)₂]⁺, [PF₆]⁻ ;
   - [CH₃-(C₆H₄)-I-(C₆H₄)-CH₂CH(CH₃)₂]⁺, LPF₆]⁻;
   - [(C₁₂H₂₅- C₆H₄)₂I]⁺, [PF₆]⁻ ; ou
- les sels de ferrocénium de ces différents anions.

Dans le cadre de l'invention, le matériau obtenu après réticulation présente une stabilité chromatique, une bonne mécanique, une bonne élasticité et une bonne résistance à la compression.

Outre les charges de renforcement, des pigments peuvent être utilisés pour teinter la composition dentaire selon l'utilisation envisagée et les groupes ethniques.

Par exemple, on utilise des pigments rouges en présence de microfibres pour les compositions dentaires utilisées pour la préparation de prothèses dentaires afin de simuler les vaisseaux sanguins.

On emploie aussi des pigments à base d'oxydes métalliques (oxydes de fer et/ou titane et/ou aluminium et/ou zirconium, etc.) pour les compositions dentaires utilisées pour la préparation de matériau de restauration, afin d'obtenir un matériau réticulé de couleur ivoire.

D'autres additifs peuvent être incorporés au sein des compositions dentaires selon l'invention. Par exemple, des biocides, des stabilisants, des agents de flaveur, des plastifiants et des promoteurs d'adhérence.

Parmi les additifs envisageables, on utilisera avantageusement des co-réactifs réticulables et/ou polymérisables de type organique. Ces co-réactifs sont liquides à température ambiante ou thermofusibles à température inférieure à 100°C, et chaque co-réactif comprend au moins deux fonctions réactives tels que oxétane-alcoxy, oxétane-hydroxy, oxétane-alcoxysilyle, carboxy-oxétane, oxétane-oxétane, alcénylether-hydroxy, alcénylether-alcoxysilyle, époxy-alcoxy, époxy-alcoxysilyles, dioxolane-dioxolane-alcool, etc.

Les compositions dentaires selon l'invention peuvent être utilisées pour de nombreuses applications dentaires, et en particulier dans le domaine des prothèses dentaires, dans le domaine de la restauration dentaire et dans le domaine des dents provisoires.

La composition dentaire selon l'invention se présente de préférence sous la forme d'un seul produit contenant les différents composants *("monocomposant")* ce qui facilite sa mise en oeuvre, notamment dans le domaine des prothèses dentaires. Eventuellement, la stabilité de ce produit peut être assurée par des dérivés organiques à fonctions amines selon l'enseignement du document WO 98/07798.

Dans le domaine des prothèses dentaires, le produit sous la forme "monocomposant" peut être déposé à l'aide d'une seringue directement sur le modèle en plâtre ou dans une clé. Puis, il est polymérisé (polymérisation par couches successives possibles) à l'aide d'une lampe UV (spectre lumière visible 200 - 500 nm).

En général, il est possible de réaliser en 10 à 15 min une prothèse dentaire durable et esthétique.

Il est à noter que les produits obtenus à partir de la composition dentaire selon l'invention sont non poreux. Ainsi, après un éventuel polissage à l'aide d'une brosse feutre par exemple, la surface des prothèses dentaires obtenues est lisse et brillante et donc ne nécessite pas d'utilisation de vernis.

Les applications dans le domaine des prothèses dentaires sont essentiellement celles de la prothèse adjointe, que l'on peut diviser en deux types :
- prothèse totale en cas de patient complètement édenté ;
- prothèse partielle due à l'absence de plusieurs dents se traduisant par soit une prothèse provisoire, soit un appareil squeletté.

Dans le domaine de la restauration dentaire, la composition dentaire selon l'invention peut être utilisée en tant que matériau d'obturation des dents antérieures et postérieures en différentes teintes (par exemple, teintes 'VITA"), rapide et facile à mettre en oeuvre.

La composition dentaire étant non toxique et polymérisable en couches épaisses, il n'est pas indispensable de polymériser le matériau en couches successives. En général, une seule injection de la composition dentaire est suffisante.

Les préparations pour prothèses dentaires et pour matériaux de restauration sont effectuées selon les techniques usuelles du métier.

Dans le cas d'application de la composition dentaire à une dent, soit la dent peut être prétraitée avec agent mordançant puis par un primaire d'accrochage qui peut être lui même photoréticulable ou soit la composition dentaire peut être préparée en mélange avec un primaire d'accrochage avant son utilisation.

Les Exemples et Tests suivants sont donnés à titre illustratif. Ils permettent notamment de mieux comprendre l'invention et de faire ressortir certains de ses avantages et d'illustrer quelques unes de ses variantes de réalisation.

### Exemples et Tests

a) Structures

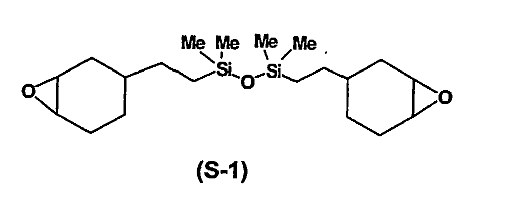

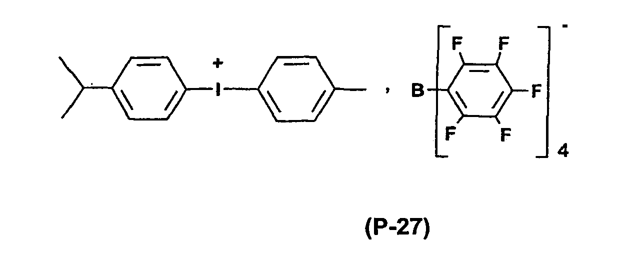

### Définition :

L'écart total de couleur ΔE représente la variation qui n'est pas due à la clarté mais aux variations de couleurs exprimées en coordonnées rectangulaires a* et b* à l'aide d'un spectrocolorimètre (modèle CIELAB).
On note Δa l'écart chromatique rouge-vert.
On note Δb l'écart chromatique jaune-bleu.
Si Δa est positif on est plus rouge.
Si Δa est négatif on est plus vert.
Si Δb est positif on est plus jaune.
Si Δb est négatif on est plus bleu.
L'écart chromatique ou différence de chromaticité Δc est exprimé par la relation
Δc=[(Δa)²+(Δb)²]^{1/2}

Les valeurs de a* mesurées pour 144 composites ont été rapportées par Inokoshi en 1996 (Bologna International Symposium) et s'échelonnent entre -5 et +4 et plus précisément entre -1 et +1. On définit arbitrairement un faible écart chromatique lorsque Δc <3 entre une mesure intitiale effectuée ¼ heure après réticulation et 5 jours après réticulation et stockage dans le noir. Le composite est photoréticulé sur une épaisseur de 2mm.
b) Les verres utilisés sont des verres commercialisés par la société Schott sous la référence G018-163 ou G018-066 contenant ou non un radiopacifiant à base d'oxyde de strontium de granulométries 0,7 µm ; 1,5µm ; 3,5µm et traités ou non par le glycidyloxytriméthoxysilane ou par le γ-méthacryloxypropyltriméthoxysilane.

Les exemples présentés ci-après décrivent le gain obtenu lorsqu'on passe d'une thioxanthone classique telle que décrite dans notre demande de brevet WO00/19967 à une thioxanthone substituée par au moins une fonction ammonium selon l'invention.

### Préparation d'une thioxanthone à fonctionnalité ammonium borate composé (V) :

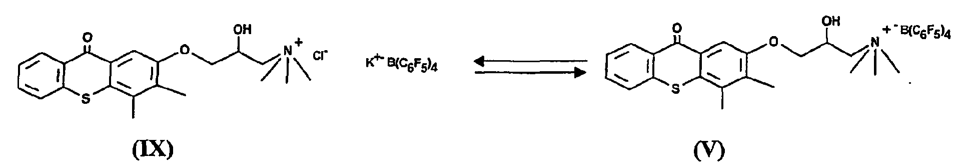

On introduit à l'abri de la lumière dans un flacon opaque 1,02 g de chlorure de 3-(3,4 dimethyl-9-oxo-9H-thioxanthen-2-yloxy)-2hydroxypropyl)triméthylammonium (vendu par la société Aldrich) ; 2,688g de sel de *« Kisbore »* KB(C₆F₅)₄ (vendu par la société Rhodia), 50ml d'isopropanol et on laisse sous agitation magnétique 48heures à température ambiante. On coule ensuite le mélange dans de l'eau déminéralisée (200ml). Un précipité jaune se forme. La suspension est filtrée sur Büchner et le solide est séché 24h à l'étuve à 100°C. On obtient le sel noté **(V)** (point de fusion 235°C ; maximum d'absorption λmax= 397,3nm).

### Préparation d'un thioxanthone à fonctionnalité ammonium borate (VI)

On introduit à l'abri de la lumière dans un flacon opaque 1,05g de chlorure de 3-(3,4 dimethyl-9-oxo-9H thioxanthen-2-yloxy)-2hydroxypropyl)trimethylammonium (Aldrich) ; 2.25g de sel de potassium tetrakis(3,5 trifluoromethylphenyl)borate (Aldrich), 50ml d'isopropanol et on laisse sous agitation magnétique 48heures à température ambiante. On coule ensuite le mélange dans de l'eau déminéralisée (200ml). Un précipité jaune se forme. La suspension est filtrée sur Büchner et le solide est séché 24h à l'étuve à 100°C. On obtient le sel noté **(VI)** (point de fusion 230°C ; maximum d'absorption λmax= 395nm).

### Exemple 1 : Préparation d'une formulation témoin 1

On charge dans un mélangeur centrifuge de la marque Hauschild 5 g de résine siloxane ayant une teneur en monomère **(S-1)** > 90% obtenue par hydrosilylation de l'oxyde de vinylcyclohexene ; 0,6g d'une solution à 4% de dispersant Dispersbyk164^{®} dans cette résine **(S-1) ;** On ajoute 0,625g de système photoamorceur dans **(S-1)** renfermant 30% de photoamorceur **(P-27)** et 0,23% de photosensibilisateur à base de chloropropoxythioxanthone **(CPTX)** commercialisé par la société Lambson, le tout en solution dans la résine **(S-1)** sans solvant.

On agite 16s avec le mélangeur centrifuge à 3000t/min à température ambiante puis on ajoute 13,25 g de quartz (granulométrie 1.5 µm, commercialisé par la société Schott sous la référence G018-163 UF1.5 traité par 2% de glycidyltriméthoxysilane).

On agite 16s avec le mélangeur centrifuge à 3000t/min et on rajoute 1,5g de trifluorure d'ytterbium.

On agite de nouveau 16s avec le mélangeur centrifuge à 3000t/min puis on ajoute1,5 g de silice de combustion (SiO₂>99%) commercialisée par la société Degussa sous la référence OX50^{®} puis on agite 16s. On rajoute enfin 2,5g de silice de combustion commercialisée par la société Degussa sous la référence R202^{®}. On agite 16s avec le mélangeur centrifuge.

Les formulations réticulent sur une épaisseur de 2 à 2,5 mm avec un temps d'irradiation qui va dépendre de la thioxanthone utilisée avec une lampe Optilux Demetron qui est généralement compris entre 30s et 1 minute.

On mesure à l'aide d'un chromamètre ou colorimètre Minolta CR200 les valeurs L*,a*,b* après 1/4 heures après réticulation sur un fond blanc et après 5 jours après réticulation.

On en déduit l'écart chromatique obtenu Δc : Δc=[(Δa)²+(Δb)²]^{1/2}

### Exemple 2 : Préparation d'une formulation selon l'invention :

On reproduit l'expérience de l'exemple 1 en remplaçant la thioxanthone témoin par les thioxanthones **(IX)** et **(V)** seules ou en association avec les dérivés :
- 9,10-dibutoxyanthracène **(PS-39),**
- phénanthrènequinone **(PS-33) ;** et
- camphorquinone **(PS-34).**

Une formulation témoin à base des composés **(PS-34)** et **(PS-39)** est aussi formulée. Dans les exemples 2e à 2h, on remplace la charge G018-066-3,5 microns par un verre G018-163 traité GLYMO (2,5%) puis par la résine **(S-1)** à 5%.

Les résultats sont consignés dans le Tableau I.

**Tableau I**

| Exemple | Photosensibilisateur (s) | Concentration(s) ppm | T=1/4 heure ; La, b | | | T=5j ; L,a,b | | | Δc |
|---|---|---|---|---|---|---|---|---|---|
| | | | L* | a* | b* | L* | a* | b* | |
| 1a (comparatif) | CPTX | 60 | 72,3 | 4,7 | 3,9 | 76,6 | 1,97 | 6,36 | 3,65 |
| 2 a(invention) | (IX) | 80 | 76,5 | 0,96 | 10,6 | 79,3 | -0,13 | 10,4 | 1,1 |
| 2b (invention) | (IX) | 220 | 74,5 | 4,14 | 14,3 | 76,7 | 3,2 | 15,3 | 1,37 |
| 2 c(invention) | (V) | 220 | 77,7 | 0,98 | 12,4 | 80,4 | -0,31 | 11,9 | 1,38 |
| 2d (invention) | (V) | 170 | 78.8 | 0,50 | 9,3 | 80,3 | -0,8 | 8,73 | 1,42 |
| 2e (invention) | (V); (PS-39) | 170; 130 | 73,4 | 0,46 | 14,16 | 76,47 | -1,0 | 13,04 | 1,84 |
| 2 f(invention) | (V); (PS-34) ; (PS-39) | 170; 100 ; 120 | 70,1 | -0,21 | 15,68 | 73,69 | -1,33 | 13,94 | 2,07 |
| 2g (invention) | (V); (PS-33) ; (PS-39) | 170;50;110 | 69,08 | 1,41 | 15,58 | 73,42 | -0,79 | 15,07 | 2,26 |
| 2 h(comparatif) | (PS-34) ; (PS-39) | 100; 160 | Cinétique insuffisante pour la réticulation en 1 minute sur 3 mm | | | | | | |

On constate que la réticulation avec une lampe de dentiste Demetron Optilux 500 des compositions dentaires formulées avec les photosensibilisateurs **(V)** ou **(IX)** seuls ou en combinaison avec d'autres photosensibilisateurs par exemple **(PS-39), (PS-34)** ou **(PS-33)** n'engendre pas de défaut de coloration (pas de phénomène de rosissement lors de l'irradiation avec une valeur chromatique a faible immédiatement après irradiation). On observe une amélioration similaire lorsque l'on utilise une thioxanthone de formule **(VI).**

L'utilisation d'une thioxanthone **(V), (VI)** ou **(IX)** selon l'invention permet de s'affranchir des problèmes de coloration mais aussi des problèmes de cinétique que l'on rencontre en utilisant uniquement la camphorquinone **(S-34)** en association avec le dérivé anthracénique **(PS-39)** (Exemple 2 Comparatif).

On montre ainsi que les thioxanthones à fonctionnalité ammonium engendrent une stabilité de coloration accrue. On observe un changement de couleur initiale rose avec le comparatif de l'exemple 1 (CPTX) même à faible taux 60ppm qui s'atténue dans le temps mais qui est encore mesurable après 5 jours (a*= 1,97) contrairement à l'utilisation des thioxanthones à fonctionnalité ammonium qui n'engendrent pas ce défaut de coloration à faible taux et permettent de façon surprenante la conservation d'une stabilité de couleur plus importante.

## Revendications

1. - Composition dentaire photopolymérisable par un rayonnement de longueur d'onde supérieure à 390 nm comprenant :
(1) au moins un composé **(A)** réactif par voie cationique ;
(2) au moins une charge dentaire **(B);**
(3) éventuellement au moins un dispersant **(C)** comprenant au moins un polymère ou copolymère organique ;
(3) au moins un photoamorceur cationique **(D) ;** et
(4) au moins un photosensibilisateur **(E)** qui est un sel d'une thioxanthone substituée par au moins un groupement G comprenant une fonction ammonium ledit photosensibilisateur **(E),** éventuellement en association avec au moins une camphorquinone, une phénanthrènequinone et/ou un anthracène substitué, a pour formule: 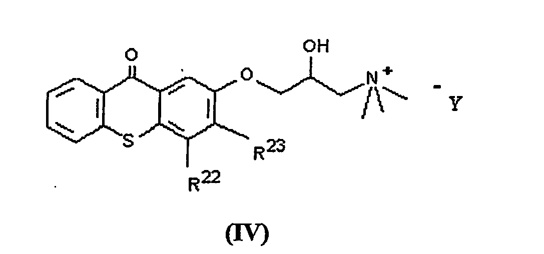 formule dans laquelle :
- R²² et R²³ sont identiques ou différents et représentent un hydrogène ou un radical alkyle en C1-C10, éventuellement substitué, de préférence R²² = R²³ = méthyle,
- (Y⁻) étant une entité anionique choisie parmi le groupe constitué par : BF₄⁻, PF₆⁻; SbF₆⁻ ; l'anion **(I)** de formule [BXₐ R_{b}]⁻ défini ci-dessous, R_{f}SO₃⁻ ; (R_{f}SO₂)₃C⁻ ou (R_{f}SO₂)₂N, avec R_{f} étant un radical alkyle linéaire ou ramifié, substitué par au moins un atome d'halogène, préférentiellement un atome de fluor,
et encore plus préférentiellement (Y⁻) est choisi parmi les borates de formules suivantes : [B(C₆H₃(CF₃)₂) ₄]⁻ et [B(C₆F₅)₄]⁻;
ledit anion **(I)** de formule [BXₐ R_{b}]⁻ étant défini de la manière suivante :
- a et b sont des nombres entiers allant pour a de 0 à 3 et pour b de 1 à 4 avec a+b=4,
- les symboles X représentent :
* un atome d'halogène (chlore, fluor) avec a = 0 à 3, ou
* une fonction OH avec a = 0 à 2,
- les symboles R sont identiques ou différents et représentent :
- un radical phényle substitué par au moins un groupement électroattracteur tel que par exemple OCF₃, CF₃, NO_{2,} CN, et/ou par au moins 2 atomes d'halogène (fluor tout particulièrement), et ce lorsque l'entité cationique est un onium d'un élément des groupes 15 à 17,
- un radical phényle substitué par au moins un élément ou un groupement électroattracteur notamment atome d'halogène (fluor tout particulièrement), CF₃, OCF₃, NO₂, CN, et ce lorsque l'entité cationique est un complexe organométallique d'un élément des groupes 4 à 10, ou
- un radical aryle contenant au moins deux noyaux aromatiques tel que par exemple biphényle, naphtyle, éventuellement substitué par au moins un élément ou un groupement électroattracteur, notamment un atome d'halogène dont le fluor en particulier, OCF₃, CF₃, NO₂, CN, quelle que soit l'entité cationique.

2. - Composition dentaire photopolymérisable par un rayonnement de longueur d'onde supérieure à 390 nm selon la revendication 1 dans laquelle le photosensibilisateur (E), éventuellement en association avec au moins une camphorquinone, une phénanthrènequinone et/ou un anthracène substitué a pour formule:

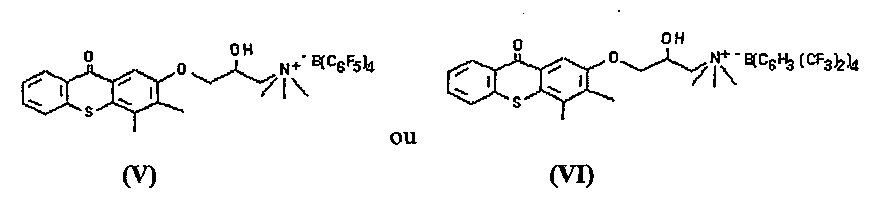

3. - Composition dentaire photopolymérisable par un rayonnement de longueur d'onde supérieure à 390 nm selon l'une quelconque des revendications précédentes **caractérisée en ce que** le photosensibilisateur **(E)** et le photoamorceur cationique **(D)** ont le même anion.

4. - Composition dentaire photopolymérisable par un rayonnement de longueur d'onde supérieure à 390 nm selon l'une quelconque des revendications précédentes, **caractérisée en ce que** le photoamorceur cationique **(D)** est de type borate et est choisi parmi ceux de formule dont :
a) l'entité cationique du borate est sélectionnée parmi :
(1) les sels d'onium de formule:
[(R¹)ₙ-A-(R²)ₘ]⁺ **(VII)**
formule dans laquelle :
- A représente un élément des groupes 15 à 17 tel que par exemple : I, S, Se, P ou N,
- R¹ représente un radical aryle carbocyclique ou hétérocyclique en C6-C20, ledit radical hétérocyclique pouvant contenir comme hétéroéléments de l'azote ou du soufre,
- R² représente R¹ ou un radical alkyle ou alkényle linéaire ou ramifié en C1-C30 ; - lesdits radicaux R¹ et R² étant éventuellement substitués par un groupement alcoxy en C1-C25, alkyle en C1-C25, nitro, chloro, bromo, cyano, carboxy, ester ou mercapto,
- m et n sont des nombres entiers, avec n + m = v + 1, v étant la valence de l'élément A,
(2) les sels d'oxoisothiochromanium ; et
(3) les sels organométalliques de formule suivante:
(L₁L₂L₃M^{)+q} **(VIII)**
formule dans laquelle :
- M représente un métal du groupe 4 à 10, notamment du fer, manganèse, chrome, cobalt,
- L1 représente 1 ligand lié au métal M par des électrons π, ligand choisi parmi les ligands η³-alkyl, η⁵- cyclopendadiènyl et η⁷-cycloheptratriènyl et les composés η⁶- aromatiques choisis parmi les ligands η⁶-benzène éventuellement substitués et les composés ayant de 2 à 4 cycles condensés, chaque cycle étant capable de contribuer à la couche de valence du métal M par 3 à 8 électrons π,
- L2 représente un ligand lié au métal M par des électrons π, ligand choisi parmi les ligands η⁷-cycloheptatriènyl et les composés η⁶-aromatiques choisis parmi les ligands η⁶- benzène éventuellement substitués et les composés ayant de 2 à 4 cycles condensés, chaque cycle étant capable de contribuer à la couche de valence du métal M par 6 ou 7 électrons π,
- L3 représente de 0 à 3 ligands identiques ou différents liés au métal M par des électrons σ, ligand(s) choisi(s) parmi CO et NO2+ ; la charge électronique totale q du complexe à laquelle contribuent L1, L2 et L3 et la charge ionique du métal M étant positive et égale à 1 ou 2 ; et
b) l'entité anionique borate a pour formule :
[BXₐ R_{b}]⁻ **(I)**
formule dans laquelle :
- a et b sont des nombres entiers allant pour a de 0 à 3 et pour b de 1 à 4 avec a+b=4,
- les symboles X représentent :
* un atome d'halogène (chlore, fluor) avec a = 0 à 3, ou
* une fonction OH avec a = 0 à 2,
- les symboles R sont identiques ou différents et représentent :
- un radical phényle substitué par au moins un groupement électroattracteur tel que par exemple OCF₃, CF₃, NO₂, CN, et/ou par au moins 2 atomes d'halogène (fluor tout particulièrement), et ce lorsque l'entité cationique est un onium d'un élément des groupes 15 à 17,
- un radical phényle substitué par au moins un élément ou un groupement électroattracteur notamment atome d'halogène (fluor tout particulièrement), CF₃, OCF₃, NO₂, CN, et ce lorsque l'entité cationique est un complexe organométallique d'un élément des groupes 4 à 10, ou
- un radical aryle contenant au moins deux noyaux aromatiques tel que par exemple biphényle, naphtyle, éventuellement substitué par au moins un élément ou un groupement électroattracteur, notamment un atome d'halogène dont le fluor en particulier, OCF₃, CF₃, NO₂, CN, quelle que soit l'entité cationique.

5. - Composition dentaire photopolymérisable par un rayonnement de longueur d'onde supérieure à 390 nm selon l'une quelconque des revendications précédentes **caractérisée en ce que** le photoamorceur cationique **(D)** est un sel d'iodonium.

6. - Composition dentaire photopolymérisable par un rayonnement de longueur d'onde supérieure à 390 nm selon l'une quelconque des revendications précédentes **caractérisée en ce que** le photoamorceur cationique **(D)** est choisi parmi le groupe constitué par les composés suivants :
**(P-16) :** [(C₈H₁₇)-O- C₆H₄-I- C₆H₅)]⁺, [B(C₆F₅)₄]⁻ ;
**(P-17) :** [C₁₂H₂₅- C₆H4-I-C₆H₅]⁺, [B(C₆F₅)₄]⁻;
**(P-18) :** [(C₈H₁₇-O- CaH₄)₂I]⁺, [B(C₆F₅)₄]⁻;
**(P-19) :** [(C₈H₁₇)-O- C₆H₄-I- C₆H₅)]⁺, [B(C₆F₅)₄]⁻;
**(P-20) :** [(C₆H₅)₂S- C₆H₄-O-C₈H₁₇]⁺, [B(C₆H₄CF₃)₄]⁻;
**(P-21) :** [(C₁₂H₂₅- C₆H₄)₂I]⁺ , [B(C₆F₅)₄]⁻;
**(P-22) :** [CH₃- C₆H₄-I- C₆H₄-CH(CH₃)₂]⁺, [B(C₆F₅)₄]⁻;
**(P-23) :** (η5 - cyclopentadiènyle) (η6 -toluène) Fe⁺, [B(C₆F₅)₄]⁻;
**(P-24) :** (η5 - cyclopentadiènyle) (η6 -methyl-1-naphtalène) Fe⁺, [B(C₆F₅)₄]⁻;
**(P-25):** (η5 - cyclopentadiènyle) (η6 - cumène) Fe⁺, [B(C₆F₅)₄]⁻;
**(P-26) :** [C₁₂H₂₅- C₆H₄)₂I]⁺ , [B(C₆H₃(CF₃)₂]⁻;
**(P-27) :** [CH₃-C₆H₄-I- C₆H₄-CH₂CH(CH₃)₂]⁺, [B(C₆F₅)₄]⁻ ;
**(P-28) :** [CH₃- C₆H₄-I- C₆H₄-CH₂CH(CH₃)₂]⁺, [B(C₆H₃(CF₃)₂)₄]⁻.; et
**(P-29) :** [CH₃- C₆H₄-I- C₆H₄-CH(CH₃)2]⁺, [B(C₆H₃(CF₃)₂)₄]⁻.

7. - Composition dentaire photopolymérisable par un rayonnement de longueur d'onde supérieure à 390 nm selon l'une quelconque des revendications précédentes **caractérisée en ce que** le composé **(A)** réactif par voie cationique est choisi dans le groupe des monomères et/ou (co)polymèreres comprenant :
les époxy, les vinyles éthers, les oxétanes, les spiroorthocarbonates, les spiro-orthoesters et leurs associations.

8. - Composition dentaire photopolymérisable par un rayonnement de longueur d'onde supérieure à 390 nm selon la revendication 7 dans laquelle le composé **(A)** réactif par voie cationique est constitué par au moins un oligomère ou polymère silicone **(A-1)** réticulable et/ou polymérisable, liquide à température ambiante ou thermofusible à température inférieure à 100 °C, et comprenant :
a) au moins un motif de formule suivante : 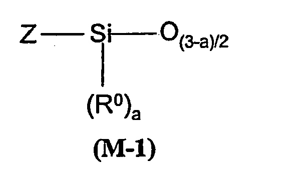 formule dans laquelle :
- a = 0, 1 ou 2,
- R⁰, identique ou différent, représente un radical alkyle, cycloalkyle, aryle, vinyle, hydrogéno, alcoxy, de préférence un alkyle inférieur en C1-C6,
- Z, identique ou différent, est un substituant organique comportant au moins une fonction réactive oxirane, alcénylether, oxétane, dioxolane et/ou carbonate, et
b) au moins deux atomes de silicium.

9. - Composition dentaire photopolymérisable par un rayonnement de longueur d'onde supérieure à 390 nm selon la revendication 8, **caractérisée en ce que** le motif **(M-1)** comprend des groupes Z choisis parmi le groupe constitué par les radicaux suivants : 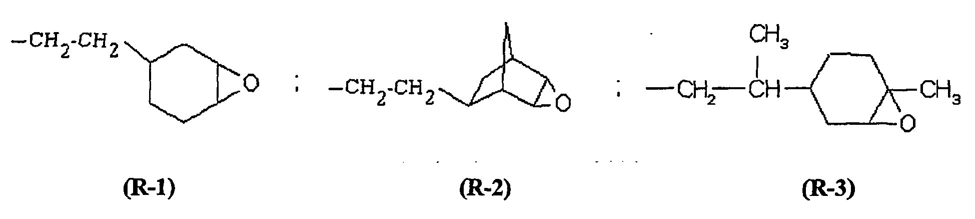 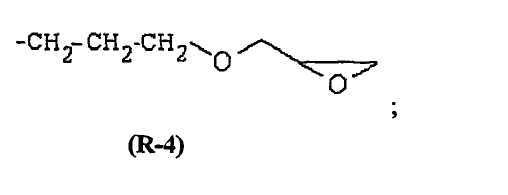 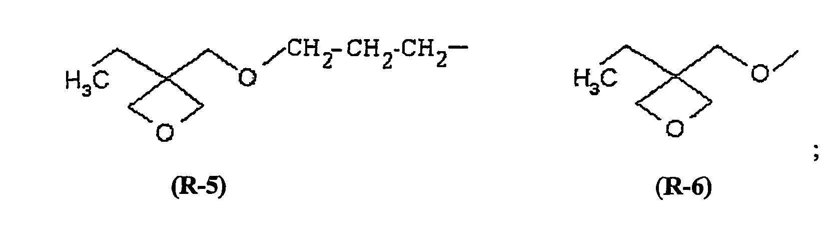 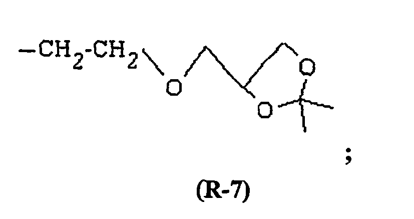
**(R-8)** -(CH₂)₃-O-CH=CH₂ ;
**(R-9)** -(CH₂)₃-O-CH=CH-R'
- avec R" représentant un radical alkyle linéaire ou ramifié en C₁-C₆.

10. - Composition dentaire photopolymérisable par un rayonnement de longueur d'onde supérieure à 390 nm selon l'une des revendications précédentes dans laquelle le composé (A) réactif par voie cationique est un silane (G-3) de formule : 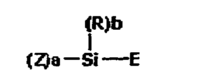 formule dans laquelle :
**E= -CH₂- ; -CH = ;** 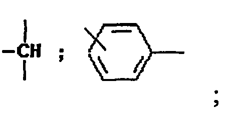
- R, identique ou différent, représente un radical alkyle, cycloalkyle, aryle, vinyle, hydrogéno, alcoxy, de préférence un alkyle inférieur en C1-C6,
- Z, identique ou différent, est un substituant organique comportant au moins une fonction oxirane, alcénylether, oxétane et/ou carbonate, et
- a+b=3.

11. - Composition dentaire photopolymérisable par un rayonnement de longueur d'onde supérieure à 390 nm selon la revendication 10 dans laquelle le silane (G-3) est choisi parmi le groupe constitué par les molécules suivantes :

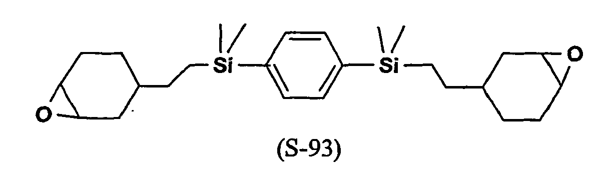

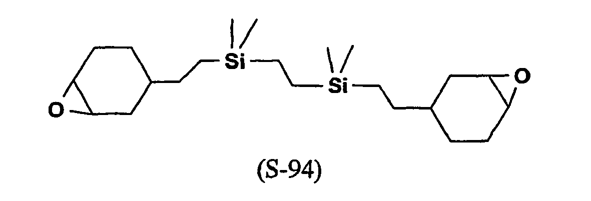

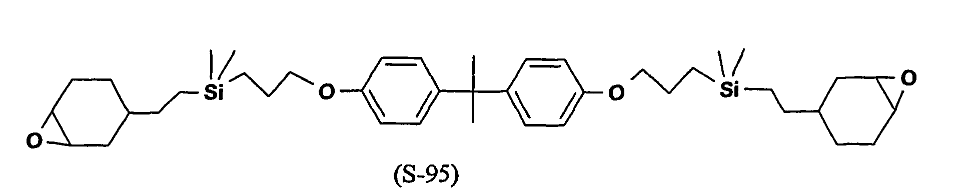

12. - Composition dentaire photopolymérisable par un rayonnement de longueur d'onde supérieure à 390 nm selon l'une des revendications précédentes dans laquelle le composé
**(A)** réactif par voie cationique est un composé organique **(G-4)** choisi parmi le groupe constitué par les molécules **(S-96)** à **(S-104) :** 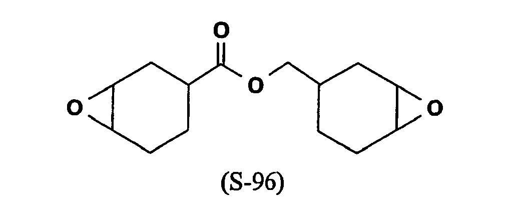 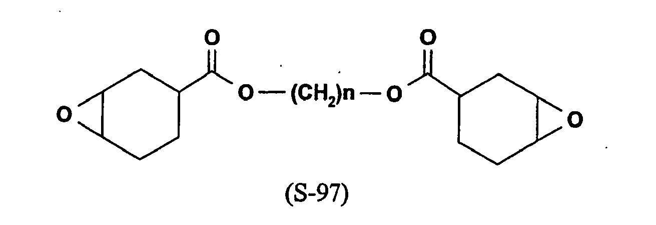 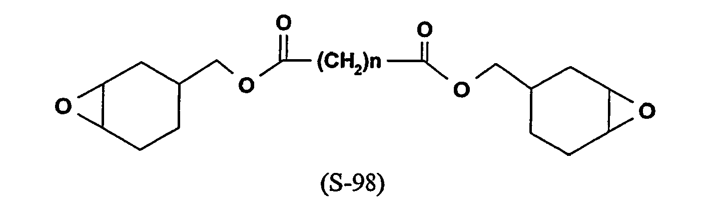 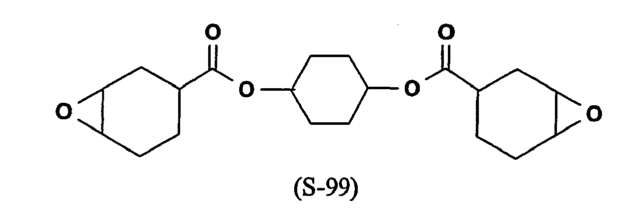 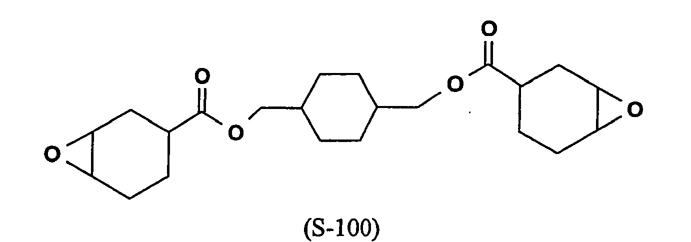 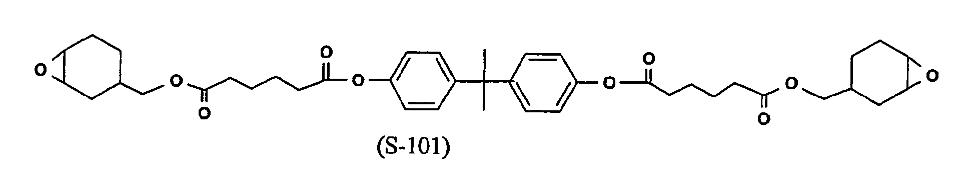 formules dans lesquelles : n est un nombre entier compris entre 1 et 10 (bornes incluses) ; 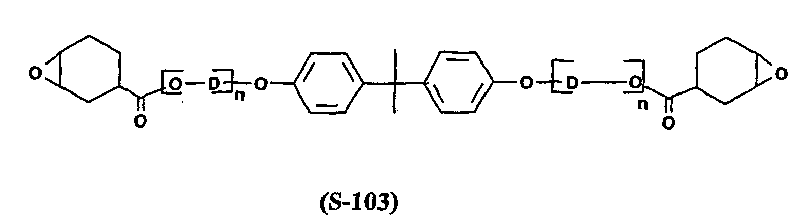 avec n<100 et D= alkyle en C1-C12 linéaires ou ramifiés. 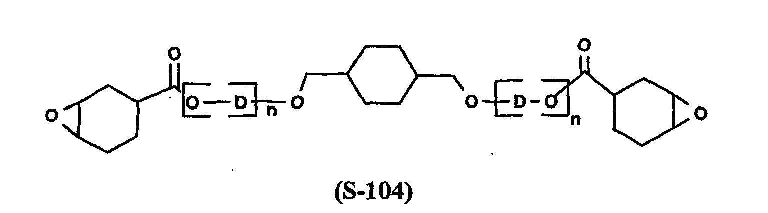 avec n<100 et le groupement D= alkyle en C1-C12 linéaires ou ramifiés.

13. - Prothèse dentaire susceptible d'être obtenue par polymérisation et/ou réticulation d'une composition selon l'une quelconque des revendications 1 à 12.

14. - Matériau de restauration dentaire susceptible d'être obtenu par polymérisation et/ou réticulation d'une composition selon l'une quelconque des revendications 1 à 12.

15. - Composé de formule :

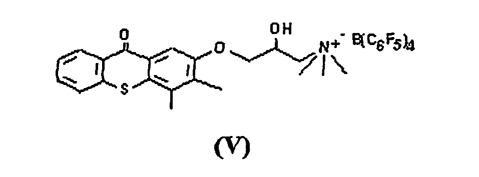

16. - Composé de formule :

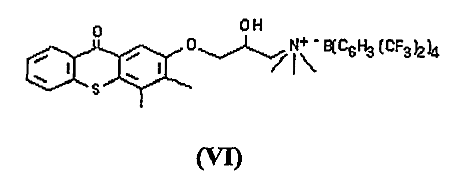

## Claims

1. Dental composition photocurable by radiation with a wavelength greater than 390 nm, comprising:
(1) at least one cationically reactive compound **(A);**
(2) at least one dental filler **(B);**
(3) optionally at least one dispersant **(C)** comprising at least one organic polymer or copolymer;
(3) at least one cationic photoinitiator **(D);** and
(4) at least one photosensitizer **(E)** which is a thioxanthone salt substituted by at least one group G containing an ammonium function; the said photosensitizer **(E),** optionally in combination with at least one camphorquinone, phenanthrenequinone and/or substituted anthracene, has the formula: 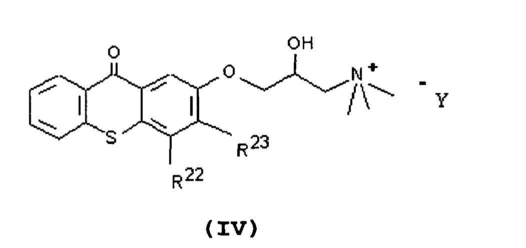 in which:
- R²² and R²³ are identical or different and represent a hydrogen or an optionally substituted C1-C10 alkyl radical; preferably R²² =R²³ = methyl,
- (Y⁻) being an anionic entity, selected from the group consisting of: BF₄⁻, PF₆⁻; SbF₆⁻; the anion **(I)** of formula [BXₐR_{b}]⁻ defined below, R_{f}SO₃⁻ ; (R_{f}SO₂) ₃C⁻ or (R_{f}SO₂)₂N⁻, where R_{f} is a linear or branched alkyl radical substituted by at least one halogen atom, preferably a fluorine atom, and even more preferably (Y⁻) is selected from the borates of the following formulae:
[B (C₆H₃ (CF₃) ₂) ₄] and [B(₆F₅)₄]⁻;
the said anion **(I)** of formula [BXₐR_{b}]⁻ being defined in the following manner:
- a and b are integers ranging from 0 to 3 for a and from 1 to 4 for b, with a + b = 4,
- the symbols X represent:
* a halogen (chlorine, fluorine) atom, with a = 0 to 3, or
* an OH function, with a = 0 to 2,
- the symbols R are identical or different and represent:
- a phenyl radical substituted by at least one electron-withdrawing group such as, for example, OCF₃, CF₃, NO₂ or CN and/or by at least two halogen atoms (especially fluorine), when the cationic entity is an onium of an element from groups 15 to 17,
- a phenyl radical substituted by at least one electron-withdrawing element or group, in particular a halogen atom (especially fluorine ) , CF₃, OCF₃, NO₂ or CN, when the cationic entity is an organometallic complex of an element from groups 4 to 10, or
- an aryl radical containing at least two aromatic nuclei, such as, for example, biphenyl, naphthyl, optionally substituted by at least one electron-withdrawing element or group, in particular a halogen atom, especially fluorine, OCF₃, CF₃, NO₂ or CN, irrespective of the cationic entity.

2. Dental composition photocurable by radiation with a wavelength greater than 390 nm according to Claim 1, wherein the photosensitizer (E), optionally in combination with at least one camphorquinone, phenanthrenequinone and/or substituted anthracene, has the formula: 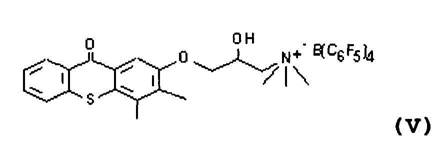 or

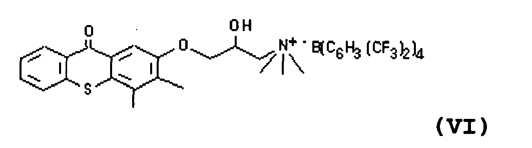

3. Dental composition photocurable by radiation with a wavelength greater than 390 nm according to either of the preceding claims, **characterized in that** the photosensitizer **(E)** and the cationic photoinitiator **(D)** have the same anion.

4. Dental composition photocurable by radiation with a wavelength greater than 390 nm according to any one of the preceding claims, **characterized in that** the cationic photoinitiator **(D)** is of borate type and is selected from those of formula for which:
a) the cationic entity of the borate is selected from:
(1) onium salts of the formula:
[(R¹)ₙ - A - (R²)ₘ]⁺ **(VII)**
in which:
- A represents an element from groups 15 to 17 such as, for example: I, S, Se, P or N,
- R¹ represents a C6-C20 heterocyclic or carbocyclic aryl radical, it being possible for said heterocyclic radical to contain nitrogen or sulfur as heteroelements,
- R² represents R¹ or a C1-C30 linear or branched alkyl or alkenyl radical, said radicals R¹ and R² being optionally substituted by a C1-C25 alkoxy, C1-C25 alkyl, nitro, chloro, bromo, cyano, carboxyl, ester or mercapto group,
- m and n are integers, with n + m = v + 1, v being the valence of the element A,
(2) oxoisothiochromanium salts; and
(3) organometallic salts of the following formula:
(L₁L₂L₃M^{)+q} **(VIII)**
in which:
- M represents a metal from group 4 to 10, especially iron, manganese, chromium or cobalt,
- L1 represents 1 ligand bonded to the metal M by n electrons, the ligand being selected from η³-alkyl, η⁵-cyclopentadienyl and η⁷⁻ cycloheptatrienyl ligands and the η⁶-aromatic compounds selected from optionally substituted η⁶ -benzene ligands and compounds having 2 to 4 fused rings, each ring being capable of contributing to the valence layer of the metal M via 3 to 8 π electrons,
- L2 represents a ligand bonded to the metal M by π electrons, the ligand being selected from η⁷-cycloheptatrienyl ligands and the η⁶-aromatic compounds selected from optionally substituted η⁶ -benzene ligands and compounds having 2 to 4 fused rings, each ring being capable of contributing to the valence layer of the metal M via 6 or 7 π electrons,
- L3 represents 0 to 3 identical or different ligands bonded to the metal M by σ electrons, the ligand(s) being selected from CO and NO2+; the total electronic charge q of the complex to which L1, L2 and L3 contribute, and the ionic charge of the metal M, being positive and being 1 or 2; and
b) the anionic borate entity has the formula:
[BXₐR_{b}]⁻ **(I)**
in which formula:
- a and b are integers ranging from 0 to 3 for a and from 1 to 4 for b, with a + b = 4,
- the symbols X represent:
* a halogen (chlorine, fluorine) atom, with a = 0 to 3, or
* an OH function, with a = 0 to 2,
- the symbols R are identical or different and represent:
- a phenyl radical substituted by at least one electron-withdrawing group such as, for example, OCF₃, CF₃, NO₂ or CN and/or by at least two halogen atoms (especially fluorine), when the cationic entity is an onium of an element from groups 15 to 17,
- a phenyl radical substituted by at least one electron-withdrawing element or group, in particular a halogen atom (especially fluorine), CF₃ , OCF₃, NO₂ or CN, when the cationic entity is an organometallic complex of an element from groups 4 to 10, or
- an aryl radical containing at least two aromatic nuclei, such as, for example, biphenyl, naphthyl, optionally substituted by at least one electron-withdrawing element or group, in particular a halogen atom, especially fluorine, OCF₃, CF₃, NO₂ or CN, irrespective of the cationic entity.

5. Dental composition photocurable by radiation with a wavelength greater than 390 nm according to any one of the preceding claims, **characterized in that** the cationic photoinitiator **(D)** is an iodonium salt.

6. Dental composition photocurable by radiation with a wavelength greater than 390 nm according to any one of the preceding claims, **characterized in that that** cationic photoinitiator **(D)** is selected from the group consisting of the following compounds:
**(P-16) :** [(C₈H₁₇)-O-C₆H₄-I-C₆H₅)]⁺, [B(C₆F₅)₄]⁻;
**(P-17) :** [C₁₂H₂₅-C₆H₄-I-C₆H₅]⁺, [B(C₆F₅)₄]⁻;
**(P-18) :** [(C₈H₁₇-O-C₆H₄)₂I]⁺, [B(C₆F₅)₄]⁻;
**(P-19) :** [(C₈H₁₇)-O-C₆H₄-I-C₆H₅)]⁺, [B(C₆F₅)₄]⁻;
**(P-20) :** [(C₆H₅)₂S-C₆H₄-O-C₈H₁₇]⁺, [B(C₆H₄CF₃)₄]⁻;
**(P-21) :** [(C₁₂H₂₅-C₆H₄)₂I]⁺, [B(C(F₅)₄]⁻;
**(P-22):** [CH₃-C₆H₄-I-C₆H₄-CH(CH₃)₂]⁺, [B(C₆F₅)₄]⁻;
**(P-23):** (η5-cyclopentadienyl)(η6-toluene)Fe⁺, [B(C₆F₅)₄]⁻;
**(P-24):** (η5-cyclopentadienyl)(η6-1-methylnaphthalene) Fe⁺, [B(C₆F₅)₄]⁻;
**(P-25) :** (η5-cyclopentadienyl) (η6-cumene)Fe⁺, [B(C₆F₅)₄]⁻;
**(P-26):** [C₁₂H₂₅-C₆H₄)₂I]⁺, [B(C₆H₃(CF₃)₂]⁻;
**(P-27) :** [CH₃-C₆H₄-I-C₆H₄-CH₂CH (CH₃)₂]⁺, [B(C₆F₅)₄]⁻;
**(P-28) :** [CH₃-C₆H₄-I-C₆H₄-CH₂CH(CH₃)₂]⁺, [B(C₆H₃(CF₃)₂)₄]⁻; and
**(P-29):** [CH₃-C₆H₄-I-C₆H₄-CH(CH₃)₂]⁺, [B(C₆H₃(CF₃)₂)₄]⁻.

7. Dental composition photocurable by radiation with a wavelength greater than 390 nm according to any one of the preceding claims, **characterized in that** the cationically reactive compound **(A)** is selected from the group of monomers and/or (co)polymers comprising:
epoxides, vinyl ethers, oxetanes, spiro-orthocarbonates, spiro-ortho-esters and combinations thereof.

8. Dental composition photocurable by radiation with a wavelength greater than 390 nm according to Claim 7, wherein the cationically reactive compound **(A)** is composed of at least one silicone oligomer or polymer **(A-1)** which is crosslinkable and/or polymerizable, is liquid at ambient temperature or thermofusible at a temperature lower than 100°C, and comprises:
a) at least one unit of the following formula: 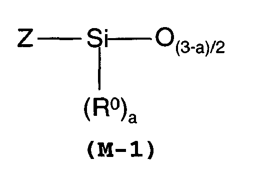 in which:
- a = 0, 1 or 2,
- R⁰, identical or different at each occurrence, represents an alkyl, cycloalkyl, aryl, vinyl, hydrogeno or alkoxy radical, preferably a C1-C6 lower alkyl,
- Z, identical or different at each occurrence, is an organic substituent containing at least one reactive oxirane, alkenyl ether, oxetane, dioxolane and/or carbonate function, and
b) at least two silicon atoms.

9. Dental composition photocurable by radiation with a wavelength greater than 390 nm according to Claim 8, **characterized in that** the unit **(M-1)** comprises groups Z selected from the group consisting of the following radicals: 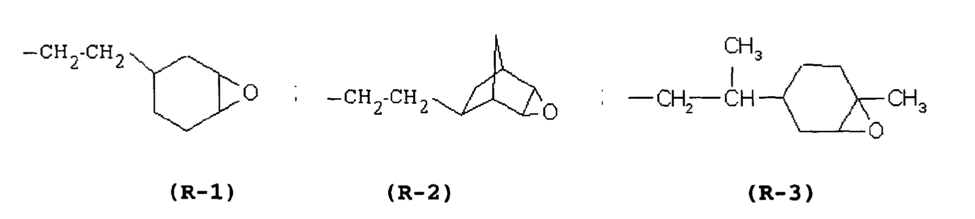 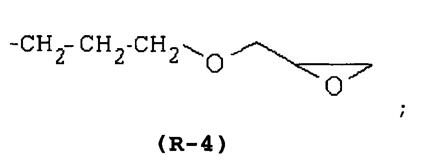 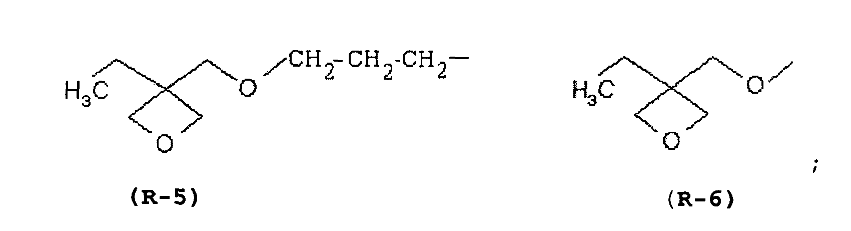 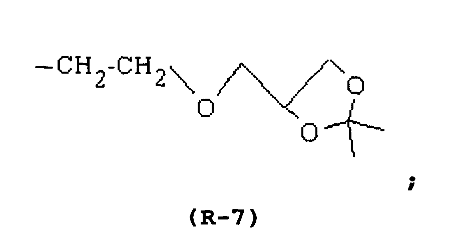 **(R-8)** -(CH₂)₃-O-CH=CH₂;
**(R-9)** -(CH₂)₃-O-CH=CH- R"
- with R" representing a C₁-C₆ linear or branched alkyl radical.

10. Dental composition photocurable by radiation with a wavelength greater than 390 nm according to one of the preceding claims, wherein the cationically reactive compound **(A)** is a silane **(G-3)** of formula: 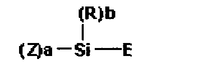 in which:
**E= -CH₂- ; -CH= ;** 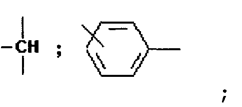
- R, identical or different at each occurrence, represents an alkyl, cycloalkyl, aryl, vinyl, hydrogeno or alkoxy radical, preferably a C1-C6 lower alkyl,
- Z, identical or different at each occurrence, is an organic substituent containing at least one oxirane, alkenyl ether, oxetane and/or carbonate function, and
- a + b = 3.

11. Dental composition photocurable by radiation with a wavelength greater than 390 nm according to Claim 10, wherein the silane **(G-3)** is selected from the group consisting of the following molecules:

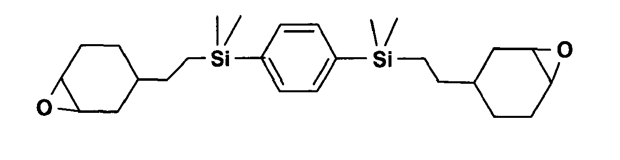

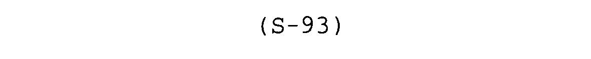

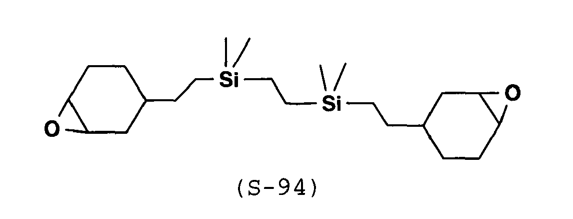

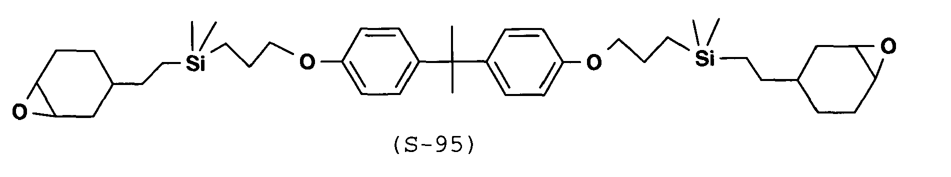

12. Dental composition photocurable by radiation with a wavelength greater than 390 nm according to one of the preceding claims, wherein the cationically reactive compound **(A)** is an organic compound **(G-4)** selected from the group consisting of the molecules **(S-96)** to **(S-104):** 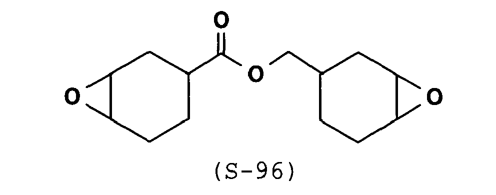 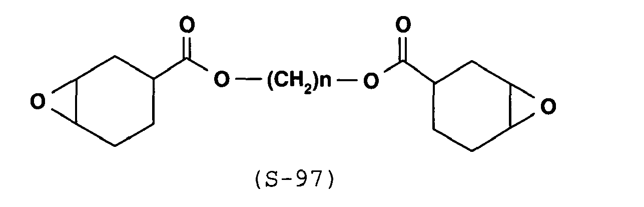 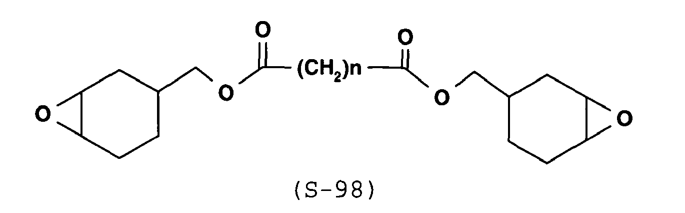 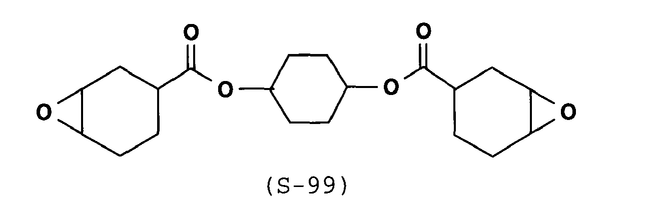 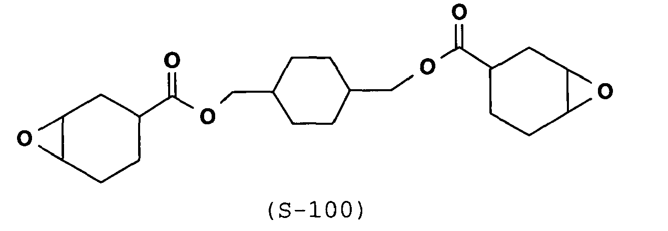 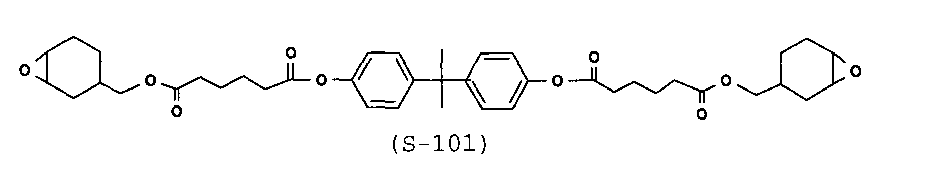 in which formulae n is an integer between 1 and 10 (inclusive); 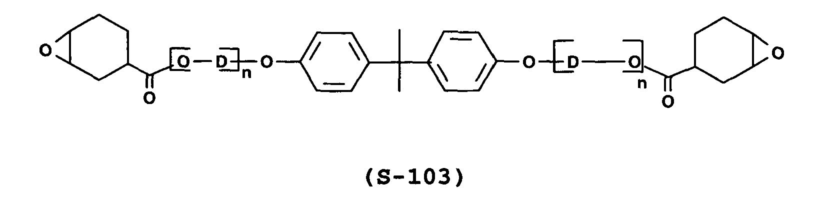 with n < 100 and D = linear or branched C1-C12 alkyl; 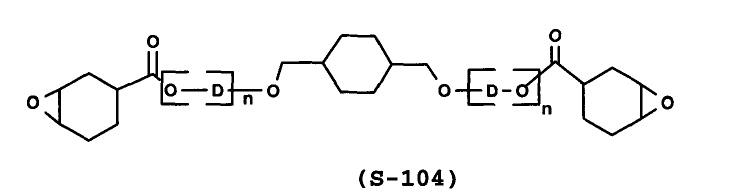 with n < 100 and the group D = linear or branched C1-C12 alkyl.

13. Dental prosthesis obtainable by polymerizing and/or crosslinking a composition according to any one of Claims 1 to 12.

14. Dental restoration material obtainable by polymerizing and/or crosslinking a composition according to any one of Claims 1 to 12.

15. Compound of the formula:

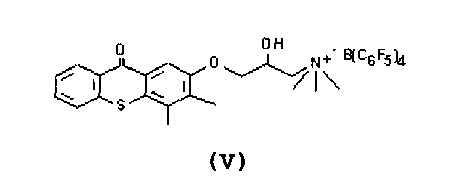

16. Compound of the formula:

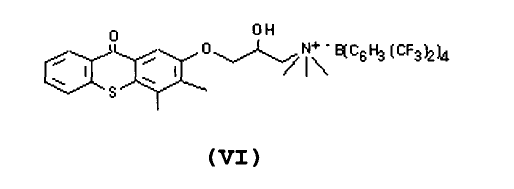

## Patentansprüche

1. Dentale Zusammensetzung, photopolymerisierbar durch eine Strahlung mit einer Wellenlänge von über 390 nm, umfassend:
(1) mindestens eine Verbindung **(A),** die auf kationischem Wege reaktiv ist,
(2) mindestens einen dentalen Füllstoff **(B);**
(3) gegebenenfalls mindestens ein Dispersionsmittel **(C),** umfassend mindestens ein organisches Polymer oder Copolymer;
(3) mindestens einen kationischen Photoinitiator **(D);** und
(4) mindestens einen Photosensibilisator **(E),** der ein Salz eines Thioxanthons ist, substituiert durch mindestens eine Gruppe G, die eine Ammonium-Funktion umfasst, wobei der genannte Photosensibilisator **(E),** gegebenenfalls in Assoziation mit mindestens einem Kampferchinon, einem Phenanthrenchinon und/oder einem substituierten Anthracen, die Formel: 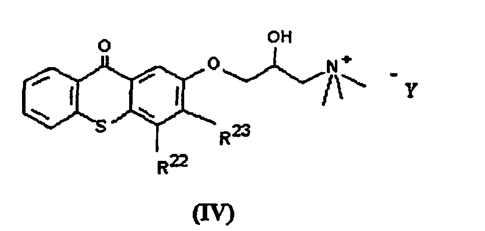 besitzt, in der
- R²² und R²³ gleich oder verschieden sind und einen Wasserstoff oder einen Rest Alkyl mit 1 bis 10 Kohlenstoffatomen, gegebenenfalls substituiert, darstellen, wobei vorzugsweise R²² = R²³ = Methyl ist,
- (Y⁻) eine anionische Einheit ist, gewählt aus der Gruppe, die gebildet wird durch: BF₄⁻, PF₆⁻; SbF₆⁻; das Anion (I) der Formel [BXₐR_{b}]⁻ wie unten definiert, R_{f}SO₃⁻; (R_{f}SO₂)₃C⁻ oder (R_{f}SO₂)₂N⁻, mit R_{f}, das ein linearer oder verzweigter Rest Alkyl ist, substituiert durch mindestens ein Halogenatom, vorzugsweise ein Fluoratom,
und ganz besonders bevorzugt wird (Y⁻) unter den Boraten der folgenden Formeln ausgewählt: [B(C₆H₃(CF₃)₂)₄]⁻ und [B(C₆F₅)₄]⁻; wobei das genannte Anion (I) der Formel [BXₐR_{b}]⁻ in der folgenden Art und Weise definiert ist:
- a und b ganze Zahlen sind, die für a von 0 bis 3 und für b von 1 bis 4 reichen, mit a + b = 4,
- die Symbole X darstellen:
* ein Halogenatom (Chlor, Fluor) mit a = 0 bis 3, oder
* eine Funktion OH mit a = 0 bis 2,
- die Symbole R gleich oder verschieden sind und darstellen:
- einen Rest Phenyl, substituiert durch mindestens eine elektronenanziehende Gruppe wie beispielsweise OCF₃, CF₃, NO₂, CN, und/oder durch mindestens zwei Halogenatome (ganz besonders Fluor), und dies, wenn die kationische Einheit ein Onium eines Elementes der Gruppen 15 bis 17 ist,
- einen Rest Phenyl, substituiert durch mindestens ein Element oder eine elektronenanziehende Gruppe, insbesondere Halogenatom (ganz besonders Fluor), CF₃, OCF₃, NO₂, CN, und dies, wenn die kationische Einheit ein organometallischer Komplex eines Elementes der Gruppen 4 bis 10 ist, oder
- einen Rest Aryl, enthaltend mindestens zwei aromatische Kerne, wie beispielsweise Biphenyl, Naphthyl, gegebenenfalls substituiert durch mindestens ein Element oder eine elektronenanziehende Gruppe, insbesondere ein Halogenatom, dabei ganz besonders Fluor, OCF₃, CF₃, NO₂, CN, wie die kationische Einheit auch sein mag.

2. Dentale Zusammensetzung, photopolymerisierbar durch eine Strahlung mit einer Wellenlänge von über 390 nm, nach Anspruch 1, in welcher der Photosensibilisator (E), gegebenenfalls in Assoziation mit mindestens einem Kampferchinon, einem Phenanthrenchinon und/oder einem substituierten Anthracen, die Formel: 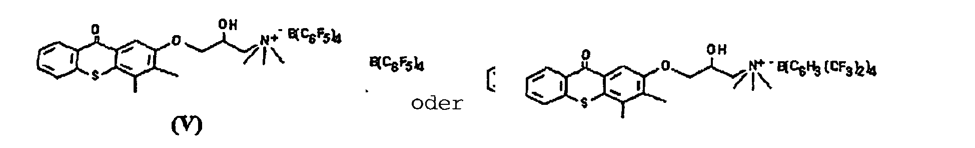 besitzt.

3. Dentale Zusammensetzung, photopolymerisierbar durch eine Strahlung mit einer Wellenlänge von über 390 nm, nach irgendeinem der vorstehenden Ansprüche, **dadurch gekennzeichnet, dass** der Photosensibilisator **(E)** und der kationische Photoinitiator **(D)** das gleiche Anion besitzen.

4. Dentale Zusammensetzung, photopolymerisierbar durch eine Strahlung mit einer Wellenlänge von über 390 nm, nach irgendeinem der vorstehenden Ansprüche, **dadurch gekennzeichnet, dass** der kationische Photoinitiator **(D)** vom Typ Borat ist und ausgewählt wird unter denen der Formel, bei der
a) die kationische Einheit des Borates ausgewählt wird unter:
(1) den Oniumsalzen der Formel
[(R¹)ₙ -A- (R²) ₘ ] ⁺ **(VII)**
in der
- A ein Element der Gruppen 15 bis 17 darstellt, wie beispielsweise: I, S, Se, P oder N,
- R¹ einen carbocyclischen oder heterocyclischen Rest Aryl mit 6 bis 20 Kohlenstoffatomen darstellt, wobei der genannte heterocyclische Rest als Heteroelemente Stickstoff oder Schwefel enthalten kann,
- R² R¹ oder einen linearen oder verzweigten Rest Alkyl oder Alkenyl mit 1 bis 30 Kohlenstoffatomen darstellt; wobei die genannten Reste R¹ und R² gegebenenfalls substituiert sind durch eine Gruppe Alkoxy mit 1 bis 25 Kohlenstoffatomen, Alkyl mit 1 bis 25 Kohlenstoffatomen, Nitro, Chlor, Brom, Cyano, Carboxy, Ester oder Mercapto,
- m und n ganze Zahlen sind, mit n + m = v + 1, wobei v die Valenz des Elementes A ist,
(2) den Oxoisothiochromanium-Salzen; und
(3) den Organometall-Salzen der folgenden Formel:
(L₁L₂L₃M)^{+q} **(VIII)**
in der
- M ein Metall der Gruppe 4 bis 10 darstellt, insbesondere Eisen, Mangan, Chrom, Cobalt,
- L1 einen Liganden darstellt, gebunden an das Metall M durch π-Elektronen, wobei der Ligand ausgewählt wird unter den Liganden η³-alkyl, η⁵-cyclopentadienyl und η⁷-cycloheptatrienyl, und den η⁶-aromatischen Verbindungen, ausgewählt unter den Liganden η⁶-Benzol, gegebenenfalls substituiert, und den Verbindungen mit 2 bis 4 kondensierten Ringen, wobei jeder Ring fähig ist, zur Abdeckung der Valenz des Metalls M durch 3 bis 8 π-Elektronen beizutragen,
- L2 einen Liganden darstellt, gebunden an das Metall M durch π-Elektronen, wobei der Ligand ausgewählt wird unter den Liganden η⁷-cycloheptatrienyl und den η⁶-aromatischen Verbindungen, ausgewählt unter den Liganden η⁶-Benzol, gegebenenfalls substituiert, und den Verbindungen mit 2 bis 4 kondensierten Ringen, wobei jeder Ring fähig ist, zur Abdeckung der Valenz des Metalls M durch 6 oder 7 π-Elektronen beizutragen,
- L3 0 bis 3 gleiche oder verschiedene Liganden darstellt, gebunden an das Metall M durch σ-Elektronen, wobei der (die) Ligand(en) ausgewählt wird (werden) unter CO und NO₂+; wobei die elektronische Gesamtladung q des Komplexes, zu dem L1, L2 und L3 beitragen, und die ionische Ladung des Metalls M positiv und gleich 1 oder 2 ist; und
b) die anionische Einheit des Borates die Formel
[BXₐR_{b}]⁻ **(I)**
besitzt, in der
- a und b ganze Zahlen sind, die für a von 0 bis 3 und für b von 1 bis 4 reichen, mit a + b = 4,
- die Symbole X darstellen:
* ein Halogenatom (Chlor, Fluor) mit a = 0 bis 3, oder
* eine Funktion OH mit a = 0 bis 2,
- die Symbole R gleich oder verschieden sind und darstellen:
- einen Rest Phenyl, substituiert durch mindestens eine elektronenanziehende Gruppe wie beispielsweise OCF₃, CF₃, NO₂, CN, und/oder durch mindestens zwei Halogenatome (ganz besonders Fluor), und dies, wenn die kationische Einheit ein Onium eines Elementes der Gruppen 15 bis 17 ist,
- einen Rest Phenyl, substituiert durch mindestens ein Element oder eine elektronenanziehende Gruppe, insbesondere Halogenatom (ganz besonders Fluor), CF₃, OCF₃, NO₂, CN, und dies, wenn die kationische Einheit ein organometallischer Komplex eines Elementes der Gruppen 4 bis 10 ist, oder
- einen Rest Aryl, enthaltend mindestens zwei aromatische Kerne, wie beispielsweise Biphenyl, Naphthyl, gegebenenfalls substituiert durch mindestens ein Element oder eine elektronenanziehende Gruppe, insbesondere ein Halogenatom, dabei ganz besonders Fluor, OCF₃, CF₃, NO₂, CN, wie die kationische Einheit auch sein mag.

5. Dentale Zusammensetzung, photopolymerisierbar durch eine Strahlung mit einer Wellenlänge von über 390 nm, nach irgendeinem der vorstehenden Ansprüche, **dadurch gekennzeichnet, dass** der kationische Photoinitiator **(D)** ein Iodoniumsalz ist.

6. Dentale Zusammensetzung, photopolymerisierbar durch eine Strahlung mit einer Wellenlänge von über 390 nm, nach irgendeinem der vorstehenden Ansprüche, **dadurch gekennzeichnet, dass** der kationische Photoinitiator **(D)** aus der Gruppe gewählt wird, die gebildet wird durch die folgenden Verbindungen:
**(P-16) :** [(C₈H₁₇)-O-C₆H₄-I-C₆H₅)]⁺, [B(C₆F₅)₄]⁻;
**(P-17):** [C₁₂H₂₅-C₆H₄-I-C₆H₅]⁺, [B(C₆F₅)₄]⁻;
**(P-18) :** [(C₈H₁₇-O-C₆H₄)₂I]⁺, [B(C₆F₅)₄]⁻;
**(P-19)** [(C₈H₁₇)-O-C₆H₄-I-C₆H₅)]⁺, [B(C₆F₅)₄]⁻;
**(P-20) :**[(C₆H₅)₂S-C₆H₄-O-C₈H₁₇]⁺, [B(C₆H₄CF₃)₄]⁻;
**(P-21):** [(C₁₂H₂₅-C₆H₄)₂-I]⁺,[B(C₆F₅)₄]⁻;
**(P-22) :** [CH₃-C₆H₄-I-C₆H₄-CH(CH₃)₂]⁺, [B(C₆F₅)₄]⁻;
**(P-23) :** (η⁵-cyclopentadienyl) (η⁶-toluol)Fe⁺ , [B(C₆F₅)₄]⁻;
**(P-24) :** (η⁵-cyclopentadienyl) (η⁶-methyl-1-naphthalin)F^{e} , [B(C₆F₅)₄]⁻;
**(P-25) :** (η⁵-cyclopentadienyl) (η⁶-cumol)Fe⁺ , [B(C₆F₅)₄]⁻;
**(P-26):** [(C₁₂H₂₅-C₆H₄)₂-I]⁺, [B(C₆H₃(CF₃)₂]⁻;
**(P-27):** [CH₃-C₆H₄-I-C₆H₄-CH₂CH(CH₃)₂]⁺ , [B(C₆F₅)₄]⁻ ;
**(P-28) :** [CH₃-C₆H₄-I-C₆H₄-CH₂CH(CH₃)₂]⁺, [B(C₆H₃(CF₃)₂)₄]⁻; und
**(P-29) :** [CH₃-C₆H₄-I-C₆H₄-CH(CH₃)₂]⁺, [B(C₆H₃(CF₃)₂)₄]⁻,

7. Dentale Zusammensetzung, photopolymerisierbar durch eine Strahlung mit einer Wellenlänge von über 390 nm, nach irgendeinem der vorstehenden Ansprüche, **dadurch gekennzeichnet, dass** die auf kationischem Wege reaktive Verbindung **(A)** aus der Gruppe von Monomeren und/oder (Co)polymeren gewählt wird, die umfasst:
die Epoxy, die Vinylether, die Oxethane, die Spiro-orthocarbonate, die Spiro-orthoester und ihre Assoziationen.

8. Dentale Zusammensetzung, photopolymerisierbar durch eine Strahlung mit einer Wellenlänge von über 390 nm, nach Anspruch 7, in der die auf kationischem Wege reaktive Verbindung **(A)** aus mindestens einem Silicon-Oligomer oder Silicon-Polymer **(A-1)** besteht, das vernetzbar und/oder polymerisierbar, bei Umgebungstemperatur flüssig oder bei einer Temperatur von unter 100 °C thermoschmelzbar ist, und umfasst:
a) mindestens eine Struktureinheit der folgenden Formel: 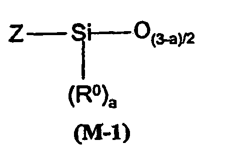 in der
- a = 0, 1 oder 2 ist,
- R°, gleich oder verschieden, einen Rest Alkyl, Cycloalkyl, Aryl, Vinyl, Hydrogeno, Alkoxy darstellt, vorzugsweise ein niederes Alkyl mit 1 bis 6 Kohlenstoffatomen,
- Z, gleich oder verschieden, ein organischer Substituent ist, der mindestens eine reaktive Funktion Oxiran, Alkenylether, Oxethan, Dioxolan und/oder Carbonat umfasst, und b)mindestens zwei Siliciumatome.

9. Dentale Zusammensetzung, photopolymerisierbar durch eine Strahlung mit einer Wellenlänge von über 390 nm, nach Anspruch 8, **dadurch gekennzeichnet, dass** die Struktureinheit **(M-1)** Gruppen Z umfasst, gewählt aus der Gruppe, die durch die folgenden Reste gebildet wird: 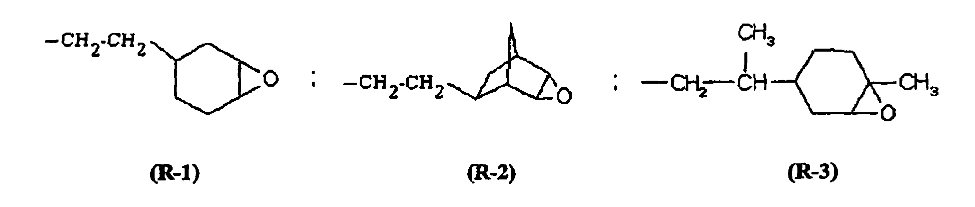 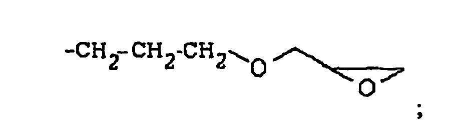 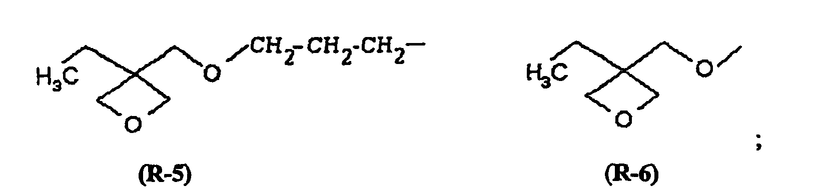
**(R-8)** -(CH₂)₃-O-CH=CH₂;
**(R-9)** -(CH₂)₃-O-CH=CH-R'
- mit R", das einen linearen oder verzweigten Rest Alkyl mit 1 bis 6 Kohlenstoffatomen darstellt.

10. Dentale Zusammensetzung, photopolymerisierbar durch eine Strahlung mit einer Wellenlänge von über 390 nm, nach einem der vorstehenden Ansprüche, in der die auf kationischem Wege reaktive Verbindung **(A)** ein Silan **(G-3)** der Formel ist, in der
**E= -CH₂- ; CH =;** 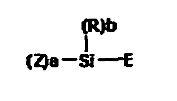 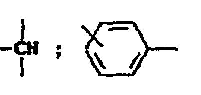 ist;
- R, gleich oder verschieden, einen Rest Alkyl, Cycloalkyl, Aryl, Vinyl, Hydrogeno, Alkoxy darstellt, vorzugsweise ein niederes Alkyl mit 1 bis 6 Kohlenstoffatomen,
- Z, gleich oder verschieden, ein organischer Substituent ist, der mindestens eine Funktion Oxiran, Alkenylether, Oxethan, und/oder Carbonat umfasst, und
- a + b = 3 ist.

11. Dentale Zusammensetzung, photopolymerisierbar durch eine Strahlung mit einer Wellenlänge von über 390 nm, nach Anspruch 10, in der das Silan **(G-3)** aus der Gruppe gewählt wird, die aus den folgenden Molekülen besteht:

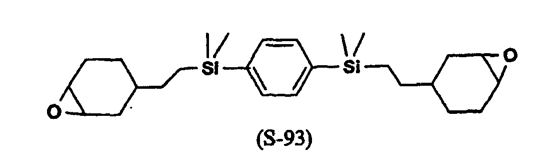

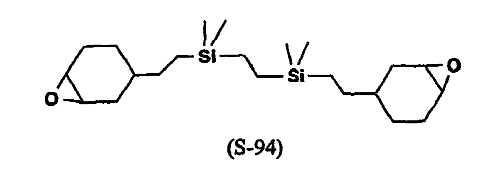

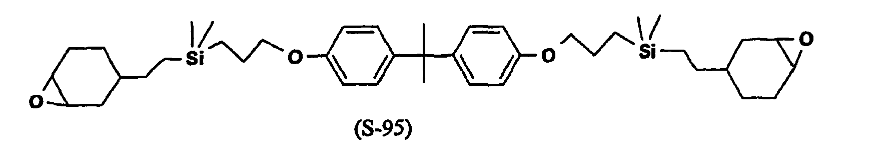

12. Dentale Zusammensetzung, photopolymerisierbar durch eine Strahlung mit einer Wellenlänge von über 390 nm, nach einem der vorstehenden Ansprüche, in der die auf kationischem Wege reaktive Verbindung **(A)** eine organische Verbindung **(G-4)** ist, gewählt aus der Gruppe, die aus den Molekülen **(S-96)** bis **(S-104)** besteht: 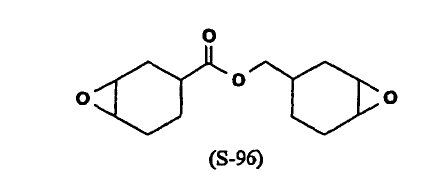 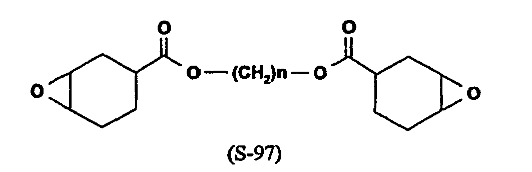 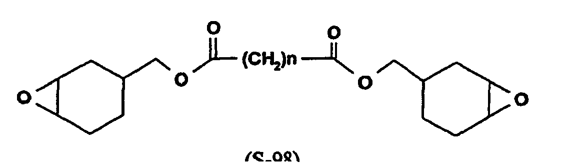 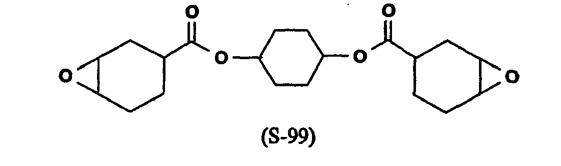 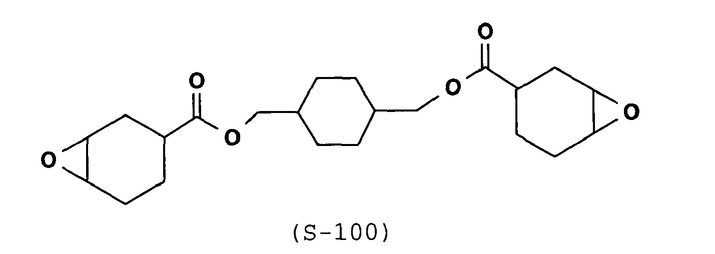 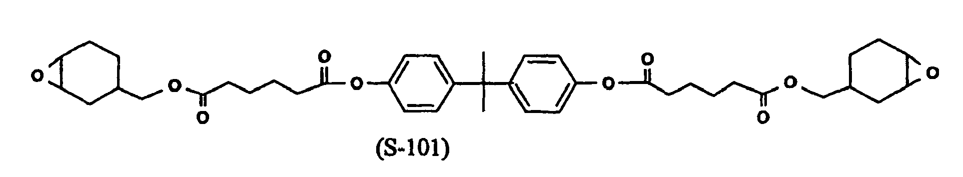 und 10 ist (Grenzwerte eingeschlossen); mit n 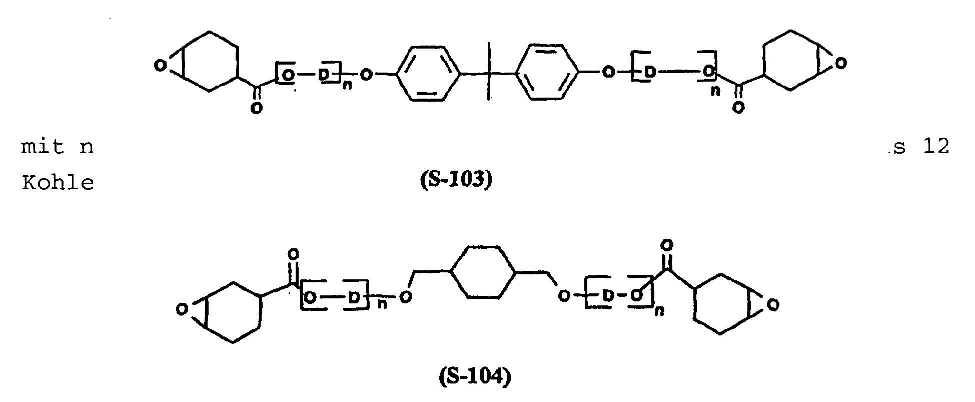 mit n < 100 und der Gruppe D = lineares oder verzweigtes Alkyl mit 1 bis 12 Kohlenstoffatomen.

13. Dental-Prothese, die geeignet ist, durch Polymerisation und/oder Vernetzung einer Zusammensetzung nach irgendeinem der Ansprüche 1 bis 12 erhalten zu werden.

14. Material zur Zahnrestaurierung, das geeignet ist, durch Polymerisation und/oder Vernetzung einer Zusammensetzung nach irgendeinem der Ansprüche 1 bis 12 erhalten zu werden.

15. Verbindung der Formel:

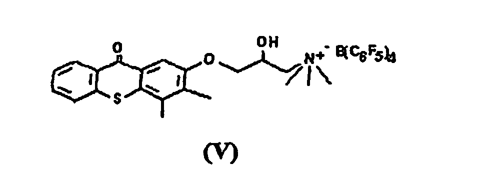

16. Verbindung der Formel:
